# EUROPEAN PATENT APPLICATION

(11) **EP 4 644 426 A1**
(43) Date of publication of application: **05.11.2025**
(21) Application number: 23911011.7
(22) Date of filing: 30.12.2023
(51) Int. Cl.: C07K 16/36, A61K 39/395, C12N 15/13, A61P 7/02

(54) **ANTIBODY AGAINST COAGULATION FACTOR XI AND/OR ACTIVATED FORM FACTOR XIA THEREOF, AND USE THEREOF**

(30) Priority: 30.12.2022 CN 202211725191
(71) Applicant: Gan & Lee Pharmaceuticals Co., Ltd., Beijing 101109 (CN)
(72) Inventor: JIAO, Jiao, Beijing 101109 (CN); LIU, Yaohui, Beijing 101109 (CN); WANG, Jiaxing, Beijing 101109 (CN); QIN, Han, Beijing 101109 (CN); FU, Shiying, Beijing 101109 (CN); XU, Xuexue, Beijing 101109 (CN); ZHANG, Jiaqi, Beijing 101109 (CN); LU, Tengfei, Beijing 101109 (CN); HAN, Xue, Beijing 101109 (CN); MA, Ziyue, Beijing 101109 (CN); JIANG, Yating, Beijing 101109 (CN)
(74) Representative: Kraus & Lederer PartGmbB
(86) International application number: PCT/CN2023/143706
(87) International publication number: WO 2024/141099

(57) **Abstract**

Disclosed are an antibody against FXI and/or FXIa or an antigen binding fragment thereof, a nucleic acid molecule encoding same, a pharmaceutical composition containing the antibody, and a use of the composition in the preparation of a drug for preventing and/or treating a disease or disorder related to coagulation or thromboembolism. Experiments show that after the humanized transformation, the antibody against FXI and/or FXIa or the antigen binding fragment thereof has a higher affinity to FXI and/or FXIa than MAA868 and can effectively inhibit the activity of FXI and/or FXIa.

## Description

### Technical Field

The present disclosure belongs to the field of biomedicine, and relates to an anti-FXI and/or FXIa antibody, and the said antibody is used for preventing and/or treating conditions involving pathological thrombosis or thromboembolism.

### Background

Coagulation, comprising humoral response and cellular response, is an important component of hemostasis, which is the process of preventing blood loss from wounds or injured sites. Pathological coagulation or thrombosis refers to the formation of blood clots that are not part of the normal hemostatic process and may lead to symptoms of disease. Examples of pathological coagulation are deep vein thrombosis, vascular graft thrombosis, coronary atherosclerotic plaque thrombosis, microvascular thrombosis, and septic diffuse intravascular coagulation. Additionally, some pathologic thrombi can be released and carried away by blood flow, occluding vessels in other parts of the body such as the lungs or brain, a process termed embolism. Diseases in which thrombosis occurs with or without embolization are collectively referred to as thromboembolic diseases.

Currently, the therapeutic drugs for thrombotic diseases mainly include three classes: anticoagulant, antiplatelet, and thrombolytic. Traditional anticoagulant drugs, such as warfarin, heparin, and low-molecular-weight heparin, and new drugs that have been launched in recent years such as FXa inhibitors (rivaroxaban, apixaban, etc.), and thrombin inhibitors (Dabigatran etexilate, hirudin, etc.), are all effective in reducing thrombus formation, but share a common limitation, i.e., the possibility of bleeding complications. For example, patients receiving anticoagulation for venous thrombosis have a risk of major bleeding of 7.2 per 100 person-years, a risk of fatal bleeding of 1.31 per 100 person-years, and a mortality rate of 13.4% due to major bleeding (Linkins LA, Ann Intern Med 2003;139:893-900). Although, the emergence of new oral anticoagulants (NOACs) such as apixaban and rivaroxaban has changed the guidelines for the use of anticoagulant, and these drugs have rapidly captured the major market for anticoagulants by virtue of a wider window of efficacy and lower risk of hemorrhage than traditional anticoagulants such as warfarin. However, bleeding caused by NOACs remains a significant clinical problem in vulnerable groups such as elderly patients or patients with renal insufficiency. Furthermore, similar to warfarin, long-term use of NOACs increases the burden of renal metabolism and leads to accelerated impairment of renal function.

Human coagulation factor XI (FXI) is an important coagulation factor of the intrinsic coagulation pathway. Research data and clinical trials have demonstrated that suppressing FXI levels to 20% through antisense oligonucleotide drugs can achieve anticoagulant effects comparable to NOACs (non-vitamin K antagonist oral anticoagulants) with a low bleeding risk. Subsequent animal studies and clinical research on human FXI (hFXI) antibodies have further confirmed that inhibiting FXI activity can simultaneously achieve effective anticoagulation with minimal bleeding.

Currently, both Bayer's FXIa antibody drug Osocimab and Novartis' FXIa antibody drug MAA868 (Abelacimab) are in Phase II clinical stage. The investigated indications include prevention of venous thromboembolism following total hip/knee replacement surgery and stroke prevention in atrial fibrillation.

The purpose of the present disclosure is to develop anti-FXI and/or FXIa antibodies with higher specificity, lower toxicity, and better clinical efficacy to provide patients with more therapeutic options.

### Summary

The disclosure has developed an antibody with excellent properties, which is able to specifically recognize FXI and/or FXIa.

In a first aspect, the disclosure provides an antibody or antigen-binding fragment thereof, which specifically binds to FXI and/or FXIa, comprising a heavy chain variable region and/or a light chain variable region, wherein:
1) the heavy chain variable region comprises:
   (i) three heavy chain complementarity determining regions (HCDRs) HCDR1, HCDR2, and HCDR3, contained within a peptide segment with an amino acid sequence of SEQ ID NO: 51, 53, 55, 57, 59, 61, 63, 65, 67, 69, 71, 73, 75, 77, 79, 81, or 83; or
   (ii) a sequence that collectively comprises at least one but no more than 5 amino acid mutations (preferably amino acid substitutions, preferably conservative substitutions) on the three heavy chain complementarity-determining regions (HCDRs), relative to the sequences of the three heavy chain complementarity-determining regions (HCDRs) defined in (i).
      and/or
2) the light chain variable region comprises:
   (i) three light chain complementarity determining regions (LCDRs) LCDR1, LCDR2, and LCDR3, contained within a peptide segment with an amino acid sequence of SEQ ID NO: 52, 54, 56, 58, 60, 62, 64, 66, 68, 70, 72, 74, 76, 78, 80, 82, or 84; or
   (ii) a sequence that collectively comprises at least one but no more than 5 amino acid mutations (preferably amino acid substitutions, preferably conservative substitutions) on the three light chain complementarity-determining regions (HCDRs), relative to the sequences of the three light chain complementarity-determining regions (HCDRs) defined in (i).

The disclosure provides an antibody or antigen-binding fragment thereof, which specifically binds to FXI and/or FXIa, comprising:
three heavy chain complementarity determining regions (HCDRs) HCDR1, HCDR2, and HCDR3, contained within a peptide segment with an amino acid sequence of SEQ ID NO: 51, 53, 55, 57, 59, 61, 63, 65, 67, 69, 71, 73, 75, 77, 79, 81, or 83; and/or
three light chain complementarity determining regions (LCDRs) LCDR1, LCDR2, and LCDR3, contained within a peptide segment with an amino acid sequence of SEQ ID NO:52, 54, 56, 58, 60, 62, 64, 66, 68, 70, 72, 74, 76, 78, 80, 82, or 84.

Optionally, the antibody or antigen-binding fragment thereof, which specifically binds to FXI and/or FXIa, comprising:
a) a heavy chain complementarity determining region HCDR3, wherein the HCDR3 comprises or consists of any one amino acid sequence selected from the group consisting of the following amino acid sequences: SEQ ID NO: 5, 11, 17, 23, 29, 35, 41 and 47, or the HCDR3 comprises an amino acid sequence having 1, 2, or 3 amino acid mutations (preferably amino acid substitutions, preferably conservative substitutions) relative to the amino acid sequences as set forth in SEQ ID NO: 5, 11, 17, 23, 29, 35, 41, and 47; and
b) a light chain complementarity determining region LCDR3, wherein the LCDR3 comprises or consists of any one amino acid sequence selected from the group consisting of the following amino acid sequences: SEQ ID NO: 8, 14, 20, 26, 32, 38, 44 and 50, or the LCDR3 comprises an amino acid sequence having 1, 2, or 3 amino acid mutations (preferably amino acid substitutions, preferably conservative substitutions) relative to the amino acid sequences as set forth in SEQ ID NO: 8, 14, 20, 26, 32, 38, 44 and 50.

Optionally, the antibody or antigen-binding fragment thereof further comprises:
c) a heavy chain complementarity determining region HCDR1, wherein the HCDR1 comprises or consists of any one amino acid sequences selected from the group consisting of the following amino acid sequences: SEQ ID NO: 3, 9, 15, 21, 27, 33, 39 and 45, or the HCDR1 comprises an amino acid sequence having 1, 2, or 3 amino acid mutations (preferably amino acid substitutions, preferably conservative substitutions) relative to the amino acid sequence as set forth in SEQ ID NO: 3, 9, 15, 21, 27, 33, 39 or 45;
d) a heavy chain complementarity determining region HCDR2, wherein the HCDR2 comprises or consists of any one amino acid sequence selected from the group consisting of the following amino acid sequences: SEQ ID NO: 4, 10, 16, 22, 28, 34, 40 and 46, or the HCDR2 comprises an amino acid sequence having 1, 2, or 3 amino acid mutations (preferably amino acid substitutions, preferably conservative substitutions) relative to the amino acid sequence as set forth in SEQ ID NO: 4, 10, 16, 22, 28, 34, 40 or 46;
e) a light chain complementarity determining region LCDR1, wherein the LCDR1 comprises or consists of any one amino acid sequence selected from the group consisting of the following amino acid sequences: SEQ ID NO: 6, 12, 18, 24, 30, 36, 42 and 48, or the LCDR1 comprises an amino acid sequence having 1, 2, or 3 amino acid mutations (preferably amino acid substitutions, preferably conservative substitutions) relative to the amino acid sequence as set forth in SEQ ID NO: 6, 12, 18, 24, 30, 36, 42 or 48; and
f) a light chain complementarity determining region LCDR2, wherein the LCDR2 comprises or consists of any one amino acid sequence selected from the group consisting of the following amino acid sequences: SEQ ID NO: 7, 13, 19, 25, 31, 37, 43, and 49, or the LCDR2 comprises an amino acid sequence having 1, 2, or 3 amino acid mutations (preferably amino acid substitutions, preferably conservative substitutions) relative to the amino acid sequence as set forth in SEQ ID NO: 7, 13, 19, 25, 31, 37, 43, or 49.

Optionally, the antibody or antigen-binding fragment thereof, comprises three heavy chain complementarity determining regions (HCDRs) HCDR1, HCDR2, and HCDR3; and three light chain complementarity determining regions (LCDRs) LCDR1, LCDR2 and LCDR3, wherein:
the HCDR1 comprises or consisting of the amino acid sequence of SEQ ID NO: 3, 9, 15, 21, 27, 33, 39, or 45, or the HCDR1 comprises an amino acid sequence having 1, 2, or 3 amino acid mutations (preferably amino acid substitutions, preferably conservative substitutions) relative to the amino acid sequence as set forth in SEQ ID NO: 3, 9, 15, 21, 27, 33, 39, or 45;
the HCDR2 comprises or consisting of the amino acid sequence of SEQ ID NO: 4, 10, 16, 22, 28, 34, 40, or 46, or the HCDR2 comprises an amino acid sequence having 1, 2, or 3 amino acid mutations (preferably amino acid substitutions, preferably conservative substitutions) relative to the amino acid sequence as set forth in SEQ ID NO: 4, 10, 16, 22, 28, 34, 40, or 46;
the HCDR3 comprises or consisting of the amino acid sequence of SEQ ID NO: 5, 11, 17, 23, 29, 35, 41, or 47, or the HCDR3 comprises an amino acid sequence having 1, 2, or 3 amino acid mutations (preferably amino acid substitutions, preferably conservative substitutions) relative to the amino acid sequence as set forth in SEQ ID NO: 5, 11, 17, 23, 29, 35, 41, or 47;
the LCDR1 comprises or consisting of the amino acid sequence of SEQ ID NO: , 12, 18, 24, 30, 36, 42, or 48, or the LCDR1 comprises an amino acid sequence having 1, 2, or 3 amino acid mutations (preferably amino acid substitutions, preferably conservative substitutions) relative to the amino acid sequence as set forth in SEQ ID NO: , 12, 18, 24, 30, 36, 42, or 48;
the LCDR2 comprises or consisting of the amino acid sequence of SEQ ID NO: 7, 13, 19, 25, 31, 37, 43, or 49, or the LCDR2 comprises an amino acid sequence having 1, 2, or 3 amino acid mutations (preferably amino acid substitutions, preferably conservative substitutions) relative to the amino acid sequence as set forth in SEQ ID NO: 7, 13, 19, 25, 31, 37, 43, or 49;
the LCDR3 comprises or consisting of the amino acid sequence of SEQ ID NO: 8, 14, 20, 26, 32, 38, 44, or 50, or the LCDR3 comprises an amino acid sequence having 1, 2, or 3 amino acid mutations (preferably amino acid substitutions, preferably conservative substitutions) relative to the amino acid sequence as set forth in SEQ ID NO: 8, 14, 20, 26, 32, 38, 44, or 50.

Optionally, the antibody or antigen-binding fragment thereof comprises:
(1) three heavy chain complementarity determining regions (HCDRs) HCDR1, HCDR2, and HCDR3, contained within a peptide segment of the amino acid sequence as set forth in SEQ ID NO: 51, and
   three light chain complementarity determining regions (LCDRs) LCDR1, LCDR2, and LCDR3, contained within a peptide segment of the amino acid sequence as set forth in SEQ ID NO: 52;
(2) three heavy chain complementarity determining regions (HCDRs) HCDR1, HCDR2, and HCDR3, contained within a peptide segment of the amino acid sequence as set forth in SEQ ID NO: 53, 67, 69, 71, 73, 75, 77, 79, or 81, and
   three light chain complementarity determining regions (LCDRs) LCDR1, LCDR2, and LCDR3, contained within a peptide segment of the amino acid sequence as set forth in SEQ ID NO: 54, 68, 70, 72, 74, 76, 78, 80, or 82;
(3) three heavy chain complementarity determining regions (HCDRs) HCDR1, HCDR2, and HCDR3, contained within a peptide segment of the amino acid sequence as set forth in SEQ ID NO: 55, and
   three light chain complementarity determining regions (LCDRs) LCDR1, LCDR2, and LCDR3, contained within a peptide segment of the amino acid sequence as set forth in SEQ ID NO: 56;
(4) three heavy chain complementarity determining regions (HCDRs) HCDR1, HCDR2, and HCDR3, contained within a peptide segment of the amino acid sequence as set forth in SEQ ID NO: 57 or 83, and
   three light chain complementarity determining regions (LCDRs) LCDR1, LCDR2, and LCDR3, contained within a peptide segment of the amino acid sequence as set forth in SEQ ID NO: 58 or 84;
(5) three heavy chain complementarity determining regions (HCDRs) HCDR1, HCDR2, and HCDR3, contained within a peptide segment of the amino acid sequence as set forth in SEQ ID NO: 59, and
   three light chain complementarity determining regions (LCDRs) LCDR1, LCDR2, and LCDR3, contained within a peptide segment of the amino acid sequence as set forth in SEQ ID NO: 60;
(6) three heavy chain complementarity determining regions (HCDRs) HCDR1, HCDR2, and HCDR3, contained within a peptide segment of the amino acid sequence as set forth in SEQ ID NO: 61, and
   three light chain complementarity determining regions (LCDRs) LCDR1, LCDR2, and LCDR3, contained within a peptide segment the amino acid sequence as set forth in SEQ ID NO: 62;
(7) three heavy chain complementarity determining regions (HCDRs) HCDR1, HCDR2, and HCDR3, contained within a peptide segment of the amino acid sequence as set forth in SEQ ID NO: 63, and
   three light chain complementarity determining regions (LCDRs) LCDR1, LCDR2, and LCDR3, contained within a peptide segment of the amino acid sequence as set forth in SEQ ID NO: 64; or
(8) three heavy chain complementarity determining regions (HCDRs) HCDR1, HCDR2, and HCDR3, contained within a peptide segment of the amino acid sequence as set forth in SEQ ID NO: 65, and
   three light chain complementarity determining regions (LCDRs) LCDR1, LCDR2, and LCDR3, contained within a peptide segment of in the amino acid sequence as set forth in SEQ ID NO: 66.

Optionally, the antibody or antigen-binding fragment thereof comprises:
(1) a HCDR1 comprising or consisting of SEQ ID NO: 3, a HCDR2 comprising or consisting of SEQ ID NO: 4, a HCDR3 comprising or consisting of SEQ ID NO: 5, a LCDR1 comprising or consisting of SEQ ID NO: 6, a LCDR2 comprising or consisting of SEQ ID NO: 7, and a LCDR3 comprising or consisting of SEQ ID NO: 8;
(2) a HCDR1 comprising or consisting of SEQ ID NO: 9, a HCDR2 comprising or consisting of SEQ ID NO: 10, a HCDR3 comprising or consisting of SEQ ID NO: 11, a LCDR1 comprising or consisting of SEQ ID NO: 12, a LCDR2 comprising or consisting of SEQ ID NO: 13, and a LCDR3 comprising or consisting of SEQ ID NO: 14;
(3) a HCDR1 comprising or consisting of SEQ ID NO: 15, a HCDR2 comprising or consisting of SEQ ID NO: 16, a HCDR3 comprising or consisting of SEQ ID NO: 17, a LCDR1 comprising or consisting of SEQ ID NO: 18, a LCDR2 comprising or consisting of SEQ ID NO: 19, and a LCDR3 comprising or consisting of SEQ ID NO: 20;
(4) a HCDR1 comprising or consisting of SEQ ID NO:21, a HCDR2 comprising or consisting of SEQ ID NO: 22, a HCDR3 comprising or consisting of SEQ ID NO: 23, a LCDR1 comprising or consisting of SEQ ID NO: 24, a LCDR2 comprising or consisting of SEQ ID NO: 25, and a LCDR3 comprising or consisting of SEQ ID NO: 26;
(5) a HCDR1 comprising or consisting of SEQ ID NO:27, a HCDR2 comprising or consisting of SEQ ID NO: 28, a HCDR3 comprising or consisting of SEQ ID NO: 29, a LCDR1 comprising or consisting of SEQ ID NO: 30, a LCDR2 comprising or consisting of SEQ ID NO: 31, and a LCDR3 comprising or consisting of SEQ ID NO: 32;
(6) a HCDR1 comprising or consisting of SEQ ID NO: 33, a HCDR2 comprising or consisting of SEQ ID NO: 34, a HCDR3 comprising or consisting of SEQ ID NO: 35, a LCDR1 comprising or consisting of SEQ ID NO: 36, a LCDR2 comprising or consisting of SEQ ID NO: 37, and a LCDR3 comprising or consisting of SEQ ID NO: 38;
(7) a HCDR1 comprising or consisting of SEQ ID NO: 39, a HCDR2 comprising or consisting of SEQ ID NO: 40, a HCDR3 comprising or consisting of SEQ ID NO: 41, a LCDR1 comprising or consisting of SEQ ID NO: 42, a LCDR2 comprising or consisting of SEQ ID NO: 43, and a LCDR3 comprising or consisting of SEQ ID NO: 44; or
(8) a HCDR1 comprising or consisting of SEQ ID NO: 45, a HCDR2 comprising or consisting of SEQ ID NO: 46, a HCDR3 comprising or consisting of SEQ ID NO: 47, a LCDR1 comprising or consisting of SEQ ID NO: 48, a LCDR2 comprising or consisting of SEQ ID NO: 49, and a LCDR3 comprising or consisting of SEQ ID NO: 50.

The disclosure further provides an antibody or antigen-binding fragment thereof, which specifically binds to FXI and/or FXIa, comprising:
a heavy chain variable region, wherein the heavy chain variable region has at least 80% identity to any one amino acid sequence selected from the group consisting of the following amino acid sequences: SEQ ID NO: 51, 53, 55, 57, 59, 61, 63, 65, 67, 69, 71, 73, 75, 77, 79, 81, and 83; preferably, the heavy chain variable region is selected from an amino acid sequence havingone or more (preferably no more than 10, more preferably no more than 5) amino acid mutations (preferably amino acid substitutions, more preferably amino acid conservative substitutions) relative to the amino acid sequence of SEQ ID NO: 51, 53, 55, 57, 59, 61, 63, 65, 67, 69, 71, 73, 75, 77, 79, 81, or 83; preferably, the amino acid mutations do not occur in the heavy chain complementarity determining regions; preferably, the heavy chain variable region has CDRs identical to those of SEQ ID NO: 51, 53, 55, 57, 59, 61, 63, 65, 67, 69, 71, 73, 75, 77, 79, 81, or 83, and the non-CDRs of the heavy chain variable region have a sequence with at least 80% identity to the non-CDRs of the amino acid sequence: SEQ ID NO: 51, 53, 55, 57, 59, 61, 63, 65, 67, 69, 71, 73, 75, 77, 79, 81, or 83; preferably, the heavy chain variable region has any one amino acid sequence selected from the group consisting of the following amino acid sequences: SEQ ID NO: 51, 53, 55, 57, 59, 61, 63, 65, 67, 69, 71, 73, 75, 77, 79, 81, and 83; and/or
a light chain variable region, wherein the light chain variable region has at least 80% identity to any one amino acid sequence selected from the group consisting of the following amino acid sequences: SEQ ID NO: 52, 54, 56, 58, 60, 62, 64, 66, 68, 70, 72, 74, 76, 78, 80, 82, and 84; preferably, the light chain variable region is selected from an amino acid sequence having one or more (preferably no more than 10, more preferably no more than 5) amino acid mutations (preferably amino acid substitutions, more preferably amino acid conservative substitutions) relative to the amino acid sequence of SEQ ID NO: 52, 54, 56, 58, 60, 62, 64, 66, 68, 70, 72, 74, 76, 78, 80, 82, and 84, preferably, the amino acid mutations do not occur in the light chain complementarity determining regions; preferably, the light chain variable region has CDRs identical to those of SEQ ID NO: 52, 54, 56, 58, 60, 62, 64, 66, 68, 70, 72, 74, 76, 78, 80, 82, or 84, and the non-CDRs of the light chain variable region have a sequence with at least 80% identity to the non-CDRs of the amino acid sequence of SEQ ID NO: 52, 54, 56, 58, 60, 62, 64, 66, 68, 70, 72, 74, 76, 78, 80, 82, or 84; preferably, the light chain variable region has any one amino acid sequence selected from the group consisting of the following amino acid sequences: SEQ ID NO: 52, 54, 56, 58, 60, 62, 64, 66, 68, 70, 72, 74, 76, 78, 80, 82, and 84.

Optionally, the antibody or antigen-binding fragment thereof comprises the heavy chain variable region and the light chain variable region, wherein the amino acid sequence pair of the heavy chain variable region and light chain variable region is selected from the group consisting of the following amino acid sequence pairs: SEQ ID NO:51 and 52, SEQ ID NO:53 and 54, SEQ ID NO:55 and 56, SEQ ID NO:57 and 58, SEQ ID NO:59 and 60, SEQ ID NO:61 and 62, SEQ ID NO:63 and 64, SEQ ID NO:65 and 66, SEQ ID NO:67 and 68, SEQ ID NO:69 and 70, SEQ ID NO:71 and 72, SEQ ID NO:73 and 74, SEQ ID NO:75 and 76, SEQ ID NO:77 and 78, SEQ ID NO:79 and 80, SEQ ID NO:81 and 82, and SEQ ID NO: 83 and 84.

Optionally, the antibody or antigen-binding fragment thereof, wherein the CDRs are defined by the IMGT definition, Kabat definition, Chothia definition, Abm definition, and/or Contact definition. Preferably, CDRs are defined by the IMGT definition or Kabat definition.

Optionally, the antibody or antigen-binding fragment thereof further comprising a constant region derived from IgG antibody, IgM antibody, IgA antibody, IgD antibody, or IgE antibody, preferably, the constant region is derived from an IgG1 antibody, an IgG2 antibody, an IgG3 antibody or an IgG4 antibody.

Optionally, the antibody or antigen-binding fragment thereof further comprises: a heavy chain constant region comprising or consisting of SEQ ID NO:85 an amino acid sequence having at least 80%, 85%, 90%, 91%, 92%, 93%, 94%, 95%, 96%, 97%, 98% or 99% identity to the sequence of SEQ ID NO:85; and/or
a light chain constant region comprising or consisting of SEQ ID NO:86 or an amino acid sequence having at least 80%, 85%, 90%, 91%, 92%, 93%, 94%, 95%, 96%, 97%, 98% or 99% identity to the sequence of SEQ ID NO:86.

Optionally, the antibody or antigen-binding fragment thereof, wherein the antibody or antigen-binding fragment thereof is a murine antibody, a chimeric antibody, a humanized antibody or a human antibody.

Optionally, the antibody or antigen-binding fragment thereof, wherein the antibody or antigen-binding fragment thereof is selected from scFv, Fab, Fab', (Fab')2, Fv fragment, disulfide-linked Fv (dsFv) and diabody.

A second aspect of the disclosure provides a pharmaceutical composition, comprising the antibody or antigen-binding fragment thereof as described in the first aspect and a pharmaceutically acceptable excipient.

Optionally, the pharmaceutical composition further comprises an additional therapeutic agent.

Optionally, the additional therapeutic agent is selected from at least one of an antiplatelet agent, an anticoagulant agent, or a thrombolytic agent, preferably, the additional therapeutic agent is selected from at least one of aspirin, clopidogrel, prasugrel, ticagrelor, abciximab, eptifibatide, vorapaxar, unfractionated heparin, heparin, low molecular weight heparin, warfarin, fondaparinux sodium, edoxaban, betrixaban, rivaroxaban, apixaban, dabigatran etexilate, argatroban, bivalirudin, streptokinase, urokinase, alteplase, and prourokinase.

Optionally, the additional therapeutic agent is another antibody or nucleic acid recognizing a different epitope of FXI and/or FXIa, or an antibody that specifically binds to a receptor or ligand selected from thrombin, Antiplasmin, Factor XII, Factor VIII, Factor VII, Factor X, Factor IX, Factor II, Tissue Factor, P-selectin and its ligand, L-selectin and its ligand, or any combination of above antibodies.

A third aspect of the disclosure provides a nucleic acid, wherein the nucleic acid has a nucleotide sequence encoding the antibody or antigen-binding fragment thereof as described in the first aspect.

A fourth aspect of the disclosure provides an expression vector, wherein the expression vector comprises the nucleic acid as described in the third aspect.

A fifth aspect of the disclosure provides a host cell, wherein the host cell comprises the expression vector as described in the fourth aspect.

Optionally, the host cell is a prokaryotic cell or a eukaryotic cell.

Optionally, the host cell is *Escherichia coli,* a yeast cell, an insect cell, a plant cell or a mammalian cell.

Optionally, the host cell is a Chinese hamster ovary cell (CHO), a CHO cell variant, a 293 cell, or an NSO cell.

A sixth aspect of the disclosure provides a method for manufacturing an antibody or antigen-binding fragment thereof, comprising culturing the host cell as described in the fifth aspect, and recovering the antibody or antigen-binding fragment thereof expressed by the cells from the culture.

A seventh aspect of the disclosure provides use of the antibody or antigen-binding fragment thereof as described in the first aspect in the preparation of a medicament for the treatment of coagulation or thromboembolism.

The disclosure further discloses the antibody or antigen-binding fragment thereof as described in the first aspect, for use in the treatment of coagulation or thromboembolism.

The disclosure further discloses a method of treating coagulation or thromboembolism, comprising administering a therapeutically effective amount of the antibody or antigen-binding fragment thereof as described in the first aspect thereof.

### Definition

For a better understanding of the present disclosure, definitions and explanations of relevant terms are provided below.

The term "antibody" or "Ab" usually refers to a Y-shaped tetrameric protein comprising two heavy (H) and two light (L) polypeptide chains held together by covalent disulfide bonds and non-covalent interactions. The light chains of antibodies can be divided into κ and λ light chains. The heavy chains can be divided into µ, δ, γ, α, and ε, which define antibody isotypes as IgM, IgD, IgG, IgA, and IgE, respectively. An Antibody also has different antibody isotypes, for example, IgG (e.g., IgG1, IgG2, IgG3, or IgG4 subtypes), IgA1, IgA2, IgD, IgE, or IgM antibodies. Each heavy chain comprises a heavy chain variable region (VH) and a heavy chain constant region (CH). The heavy chain constant region comprises 3 structural domains (CH1, CH2 and CH3). Each light chain comprises a light chain variable region (VL) and a light chain constant region (CL). The VH and VL regions can be further divided into highly variable regions (called complementary decision regions (CDR)) spaced apart by relatively conserved regions called framework regions (FR). Each VH and VL comprises three CDRs and four FRs in the following order: from the N-terminal to the C-terminal FR1, CDR1, FR2, CDR2, FR3, CDR3, and FR4, wherein the three CDRs for the VHs are HCDR1, HCDR2, and HCDR3, and the three CDRs for the VLs are LCDR1, LCDR2, and LCDR3. The variable regions (VH and VL) of each heavy chain /light-light chain pair form antigen-binding sites, respectively.

The term "antigen-binding portion" or "antigen-binding fragment" of an antibody, which can be used interchangeably in the context of this application, refers to one or more fragments of an antibody that retain the ability to bind specifically to an antigen. It has been demonstrated that the antigen-binding function of an antibody can be achieved by certain fragments of a full-length antibody. In some conditions, antigen-binding fragments include Fab, Fab', F(ab')2, Fd, Fv, dAb and complementary determining region (CDR) fragments, single-chain antibodies (e.g., scFv), chimeric antibodies, diabody, and such polypeptides comprising at least a portion of an antibody that is sufficient to confer antigen-binding capacity specific to the polypeptide. Antigen-binding fragments of antibodies can be obtained from a given antibody by conventional techniques known to those skilled in the art (e.g., recombinant DNA techniques or enzymatic or chemical cleavage methods) and can be screened for specificity in the same manner as intact antibodies.

Different analyses may be used to identify or roughly estimate the CDR region. Examples of said methods include but are not limited to, the IMGT definition, the Kabat definition, the Chothia definition, the AbM definition, and the contact definition. The IMGT definition is the intemational ImMunoGeneTics information(IMGT) (LaFranc et al., 2005. Nucl Acids Res. 33: D593-D597), which has developed into a widely adopted universal numbering system currently. The Kabat definition is the immunoglobulin comparison and numbering system proposed by Elvin A. Kabat (see, e.g., Kabat et al., Sequences of Proteins of Immunological Interest, 5th Ed. Public Health Service, National Institutes of Health, Bethesda, Md., 1991 or e.g., Johnson & Wu, Nucleic Acids Res., 28: 214-8(2000)), is a standard for numbering residues in antibodies and is commonly used to identify CDR regions. The Chothia Definition is similar to the Kabat Definition, but the Chothia Definition takes into account the location of certain structural loop regions, is a system of immunoglobulin numbering proposed by Chothia et al as a classical rule for identifying CDR boundaries based on the location of structural loop regions, see e.g. Chothia et al, J. Mol. Biol., 196:901-17 (1986); Chothia et al, Nature, 342:877-83 (1989). The AbM definition uses an integrated set of computer programs mimicking the structure of the antibody produced by the Oxford Molecular Group . See, for example, Martin et al, Proc Natl Acad Sci (USA), 86: 9268-9272 (1989); "AbMTM, A Computer Program for Modeling Variable Regions of Antibodies" Oxford, UK; Oxford Molecular, Ltd. The AbM definition uses known databases and the ab initio method to model the tertiary structure of antibodies from primary sequences, said method, for example, the method described in the following literature: Samudrala et al, "Ab Initio Protein Structure Prediction Using a Combined Hierarchical Approach" PROTEINS, Structure, Function and Genetics Suppl., 3: 194-198 (1999). The contact definition is based on an analysis of the crystal structure of a valid complex. See, e.g., MacCallum et al, J. Mol. Biol., 5: 732-45 (1996).

The term "monoclonal antibody" or "mAb" refers to an antibody molecule comprising a single molecule or its formulation. Monoclonal antibodies exhibit single binding specificity and affinity for a specific epitope.

The term "murine antibody" refers to an antibody obtained by fusing immunized mouse B cells with myeloma cells, afterward screening for murine hybrid fusion cells capable of both unlimited growth and antibody secretion, and then screening, antibody preparation, and antibody purification; or, as B cells differentiate and proliferate after antigen invasion into the mouse body and form plasma cells, plasma cells can produce and secrete antibodies.

The term "chimeric antibody" refers to an antibody in which the variable region sequence is derived from one species and the constant region sequence is derived from another species, such as an antibody in which the variable region sequence is derived from a mouse antibody and the constant region sequence is derived from a human antibody.

The term "humanized antibody" refers to an antibody in which CDR sequences derived from a germline of another mammalian species, such as mouse, have been grafted onto a human framework sequence. Additional framework region modifications can be made within the human framework sequence.

The term "secondary antibody" refers to a second antibody that binds to the antibody, i.e., an antibody to the antibody, and whose main function is to detect the presence of the antibody and amplify the signal of the primary antibody. A secondary antibody is to use antigenic properties of Macromolecular protein of an antibody to immunize a heterologous animal, and is an immunoglobulin produced by the immune system of a heterologous animal that targets the antibody. Secondary antibodies are reactive against all antibodies (e.g., IgG, IgM, or IgA, etc.) of a particular species (e.g., mouse).

The term "amino acid mutation" refers to an amino acid substitution, insertion, deletion or modification. Specifically, a mutation between a peptide and its variant at one or more amino acid sites on a peptide fragment, wherein the variant may be obtained by substitution, insertion, deletion or modification of amino acids at one or more sites on the peptide.

The term "conservative substitution" means an amino acid substitution that does not adversely affect or alter the intended properties of the protein/polypeptide comprising the amino acid sequence, whereby the variant of the antibody obtained by conservatively substituting the amino acid sufficiently retains the biological activity of its source sequence. For example, conservative substitutions may be introduced, for example, by standard techniques known in the art such as targeted mutagenesis and PCR-mediated mutagenesis. Conservative amino acid substitutions include substitutions in which an amino acid residue is replaced with an amino acid residue having a similar side chain, such as substitutions with a residue that is physically or functionally similar to the corresponding amino acid residue (e.g., having a similar size, shape, charge, chemical properties including the ability to form covalent or hydrogen bonds, etc.). Families of amino acid residues having similar side chains have been defined in the art. These families include amino acid residues having basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Thus, it is preferred to replace the corresponding amino acid residue with another amino acid residue from the same side chain family.

The terms "FXI protein", "FXI antigen", "coagulation factor XI" and "FXI" are used interchangeably and refer to different species of coagulation factor XI protein. The terms "FXla protein", "FXIa antigen", coagulation factor XI activated form of factor XIa and "FXIa" are used interchangeably and refer to different species of activated coagulation factor XI protein.

The terms "FXI" and "FXIa" include, respectively, natural FXI and FXIa proteins, and mutants and variants of natural FXI and FXIa proteins (the mutants and variants have amino acid sequences that are substantially identical to the natural primary structure).

The term "affinity" refers to the strength of the sum of the non-covalent interactions between individual binding sites of a molecule (e.g., an antibody) and its binding partner (e.g., an antigen). Unless otherwise specified, as used herein, "affinity" refers to the inherent binding affinity of a 1:1 interaction between members of a reaction-binding pair (e.g., antibody and antigen). The affinity of molecule X for its partner Y can usually be expressed by an equilibrium dissociation constant (KD), a dissociation constant (Kd), or a association constant (Ka). The affinity can be measured by general methods known in the art, including those described herein, preferably such as analysis using a Biacore^{™} system.

The term "vector" refers to a nucleic acid medium into which polynucleotides can be inserted. When the vector allows expression of the protein encoded by the inserted polynucleotide, the vector is called an expression vector. The vector may allow expression of the carried genetic material elements in the host cell by transformation, transduction or transfection into the host cell. Vectors are well known to the skilled in the art and include but are not limited to, plasmids, phages, artificial chromosomes such as yeast artificial chromosomes, bacterial artificial chromosomes, viruses; phages such as λ phage or M13 phage. Animal viruses that may be used as vectors include but are not limited to, retroviruses (including lentiviruses), adenoviruses, adeno-concomitant viruses, herpesviruses (e.g., herpes simplex viruses), poxviruses, baculoviruses, papillomaviruses, and lactopolyhedroviruses (e.g., SV40). The vector may comprise a plurality of elements for controlling expression, including but not limited to, promoter sequences, transcription start sequences, enhancer sequences, selection elements, and reporter genes. Alternatively, the vector may comprise a replication start.

The term "host cell" refers to a cell system that can be engineered to produce a target protein, protein fragment or peptide. Host cells include but are not limited to, cultured cells, such as mammalian cultured cells derived from rodents (rats, mice, guinea pigs, or hamsters), such as CHO, BHK, NS0, SP2/0, YB2/0; or human tissues or hybridoma cells, yeast cells, and insect cells, as well as cells comprised within a transgenic animal or cultured tissue. The term covers not only specific subject cells, but also the progeny of such cells. Due to certain modifications may occur in subsequent generations as a result of mutations or environmental influences, such progeny may differ from the parental cells, but are still covered by the term "host cell".

The term "transfection" refers to the uptake of foreign or exogenous DNA by a cell, the cell is transfected when said exogenous DNA is introduced into the cell membrane. Various transfection techniques are well known in the art. See, e.g., Graham et al. 1973, Virology 52:456; Sambrook et al. 2001, Molecular Cloning: A Laboratory Manual; Davis et al. 1986, Basic Methods in Molecular Biology. Biology, Elsevier; Chu et al. 1981, Gene 13: 197. The said techniques may be used to introduce one or more exogenous DNA fractions into a suitable host cell.

The term "identity" or "homology" refers to sequence similarity between two nucleotide sequences or between two polypeptides. When positions in two comparative sequences are occupied by the same base or amino acid monomer subunit, for example, if each position of two DNA molecules is occupied by an adenine, then said molecules are homologous at that position. The percentage of homology between two sequences is a function of the number of matching or homologous positions shared by the two sequences divided by the total number of positions compared x 100. For example, in the case of a best comparison of sequences, if 6 out of 10 positions in the two sequences match or are homologous, then the two sequences are 60% homologous; if 95 out of 100 positions in the two sequences match or are homologous, then the two sequences are 95% homologous.

The term "treatment" includes therapeutic treatment, prophylactic treatment, and applications in reducing the risk of the subject developing a disease or other risk factors. Treatment includes not only complete cure of the disease, but also includes alleviation of symptms or mitigation of potential risks.

As used herein, the term "preventing and/or treating a disease or condition associated with coagulation or thromboembolism" may refer to one or more of the following conditions for which the anti-FXI and/or FXIa antibodies of the present disclosure, or antigen-conjugated fragments thereof, may be used to prevent or treat:
- Thromboembolism in an individual suspected or diagnosed to have an arrhythmia, such as sudden, persistent or permanent atrial fibrillation or atrial fibrillation;
- Stroke prevention in atrial fibrillation (SPAF) in a subgroup of patients with atrial fibrillation (AF) undergoing percutaneous coronary intervention (PCI);
- Treatment of acute venous thromboembolic events (VTE) and durable secondary VTE prophylaxis in patients at high bleeding risk;
- Cerebral and cardiovascular events in secondary prevention after transient ischemic attack (TIA) or non-disabling stroke and thromboembolic event prophylaxis in heart failure with concomitant sinus rhythm;
- Clot formation and thromboembolism in the left atrium in individuals undergoing cardioversion for arrhythmia;
- Thrombosis before, during, and after resection procedures for cardiac arrhythmias;
- Venous thrombosis, which includes (but is not limited to) the treatment and secondary prevention of deep or superficial venous thrombosis in the lower or upper part of the body, thrombosis of the abdominal and thoracic veins, sinus thrombosis, and jugular vein thrombosis;
- Thrombosis on any artificial surface in a vein-like catheter or pacemaker lead;
- Pulmonary embolism in patients with or without venous thrombosis;
- Chronic thromboembolic pulmonary hypertension (CTEPH);
- Arterial thrombus on ruptured atherosclerotic plaque, thrombus on intra-arterial adjuncts or catheters, and thrombus in ostensibly normal arteries, a condition that includes (but is not limited to) acute coronary syndromes, ST-elevation myocardial infarction, non-ST-elevation myocardial infarction, unstable colic, stent thrombosis, thrombus in any artificial surface in the arterial system, and thrombus in an artery of the lungs of an individual with or without pulmonary hypertension;
- Thrombosis and thromboembolism in patients undergoing percutaneous coronary intervention (PCI);
- Cardiac and cryptogenic strokes;
- Thrombosis in patients with invasive and non-invasive cancer malignancies;
- Thrombosis on indwelling catheters;
- Thrombosis and thromboembolism in patients with serious diseases;
- Cardiac thrombosis and thromboembolism, which includes, but is not exclusive to, cardiac thrombosis following myocardial infarction, cardiac thrombosis associated with conditions such as cardiac aneurysm, myocardial fibrosis, cardiac enlargement and dysfunction, myocarditis, and artificial surfaces in the heart;
- Thromboembolism in patients with heart valve disease with or without atrial fibrillation;
- Thromboembolism on valvular mechanical or biological aids;
- Thromboembolism in patients with natural or artificial cardiac patches, arterial or venous conduits following cardiac repair of simple or complex cardiac malformations;
- Venous thrombosis and thromboembolism following knee replacement surgery, hip replacement surgery and orthopedic surgery, thoracic or abdominal surgery;
- Arterial or venous thrombosis following neurosurgical procedures including intracranial and spinal cord interventions;
- Congenital or acquired thrombotic tendencies, including (but not exclusively) coagulation factor V Leiden, prothrombin mutations, antithrombin III, protein C and protein S deficiencies, coagulation factor XIII mutations, familial fibrinogen dyskinesia, congenital fibrinolysinogen deficiencies, increased levels of coagulation factor XI, sickle cell disease, antiphospholipid syndrome, autoimmune diseases, chronic intestinal disease, nephrotic syndrome, hemolytic uremic syndrome, myeloproliferative diseases, disseminated intravascular coagulation, paroxysmal nocturnal hemoglobinuria, and heparin-induced thrombocytopenia;
- thrombosis and thromboembolism in chronic kidney disease; and
- thrombosis and thromboembolism in patients undergoing hemodialysis and patients undergoing extracorporeal membrane oxidation.

The term "therapeutically effective amount" refers to the amount of antibody that, when administered to a subject for use in the treatment of at least one clinical symptom of a disease or disorder, is sufficient to produce an effect on such treatment of the disease, disorder or symptom. The therapeutically effective amount may vary with the severity of the disease or disorder, or the age of the subject to be treated and/or the weight of the subject to be treated.

The term "pharmaceutically acceptable excipient" refers to a carrier and/or excipient that is pharmacologically and/or physiologically compatible with the subject and the active agent, and is well known in the art. These include but are not limited to pH modifiers, surfactants, adjuvants and ionic strength enhancers.

### Description of the Drawings

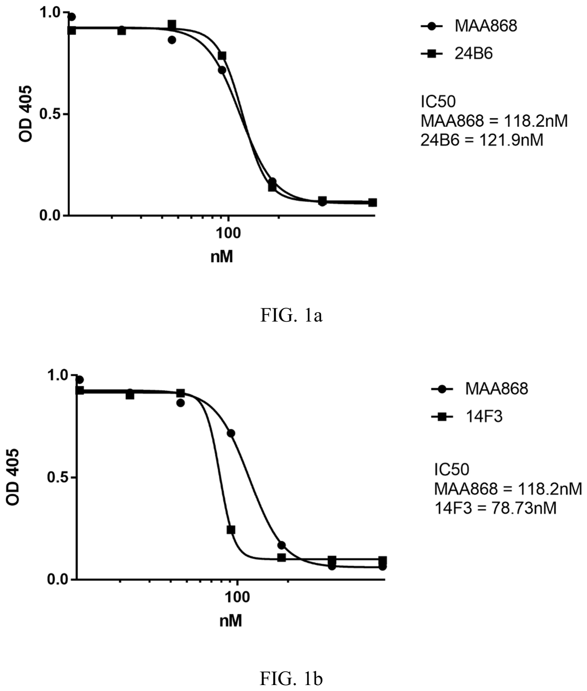

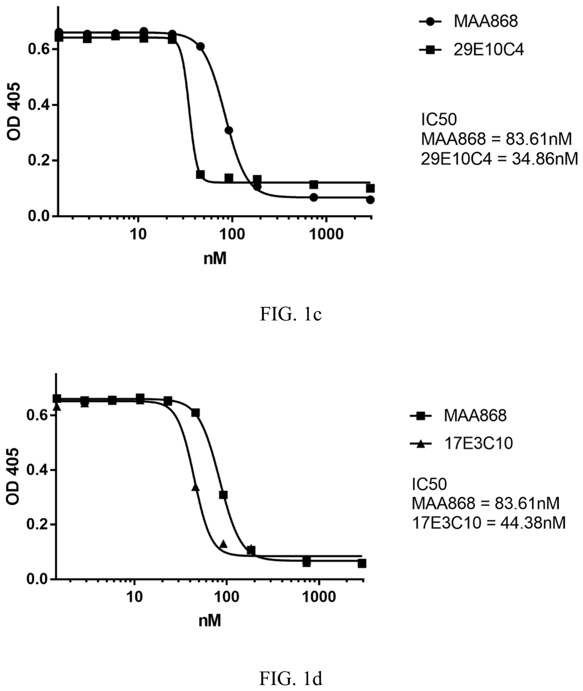

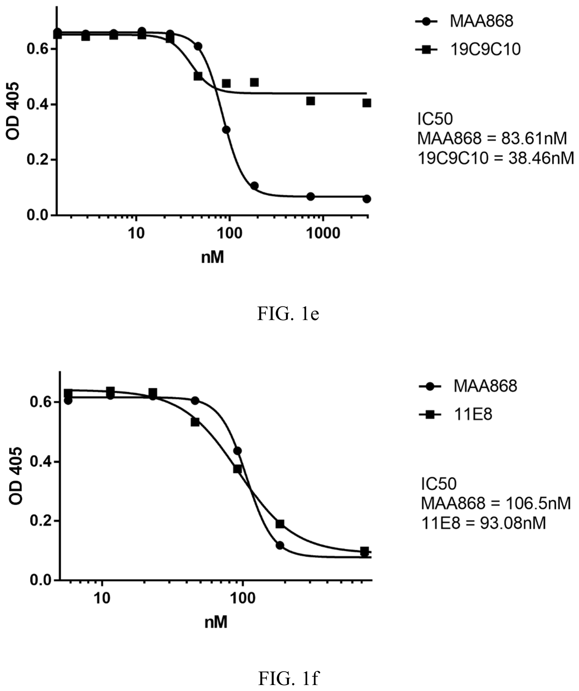

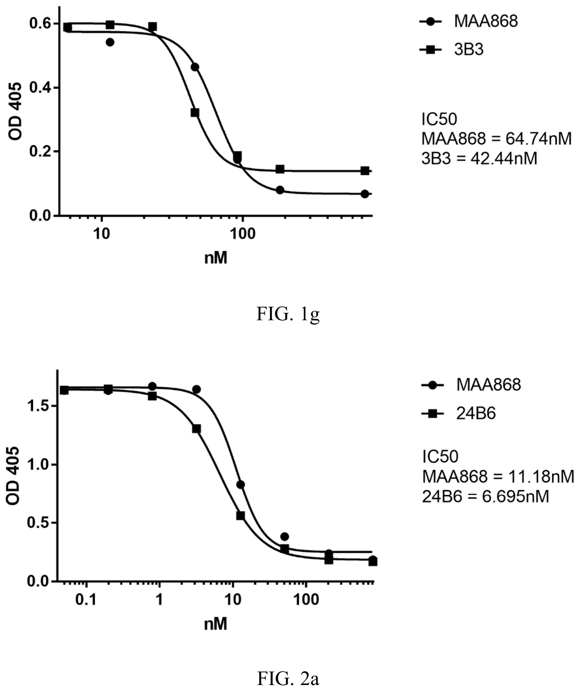

FIGs. 1a-1g show the S2366 activity assay results for chimeric antibodies 24B6, 14F3, 29E10C4, 17E3C10, 19C9C10, 11E8, and 3B3, respectively. The x-axis represents concentration (nM), and the y-axis represents absorbance at OD₄₀₅.

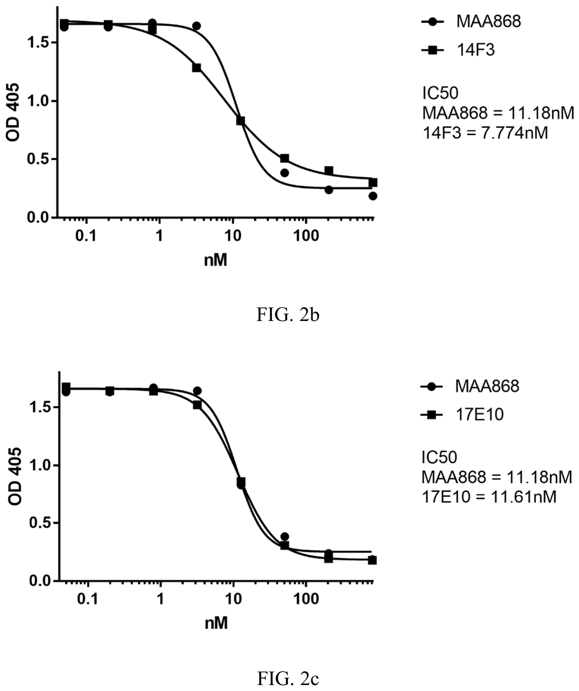

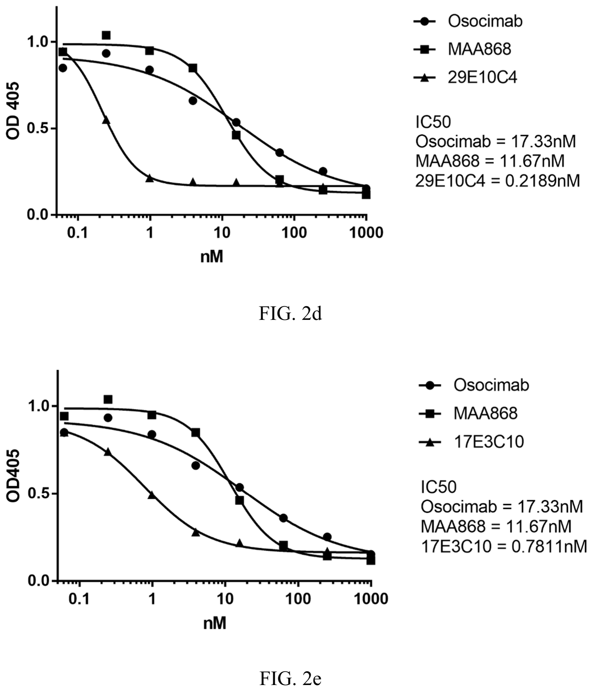

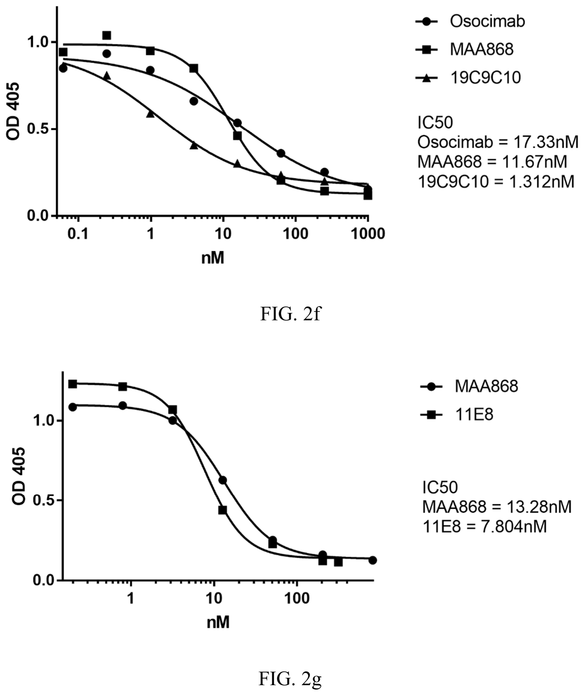

FIGs. 2a-2g show the FXIa assay kit activity results for chimeric antibodies 24B6, 14F3, 17E10, 29E10C4, 17E3C10, 19C9C10, and 11E8, respectively. The x-axis represents concentration (nM), and the y-axis represents absorbance at OD₄₀₅.

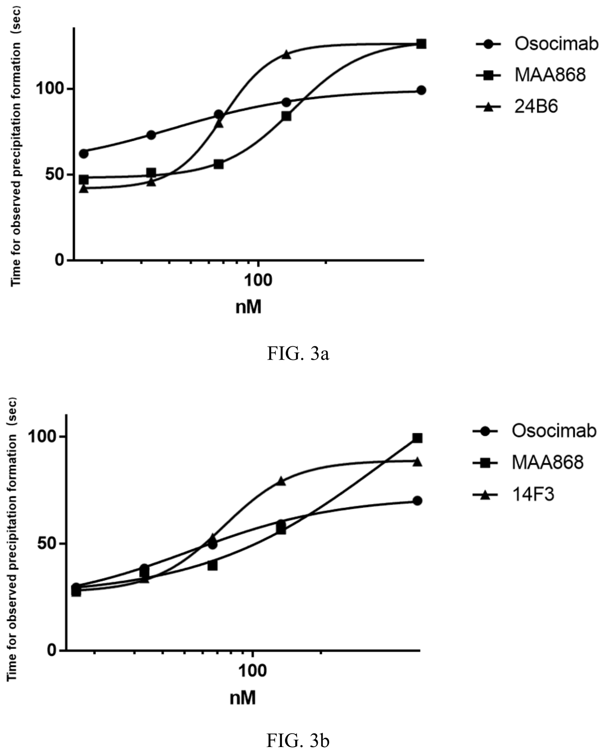

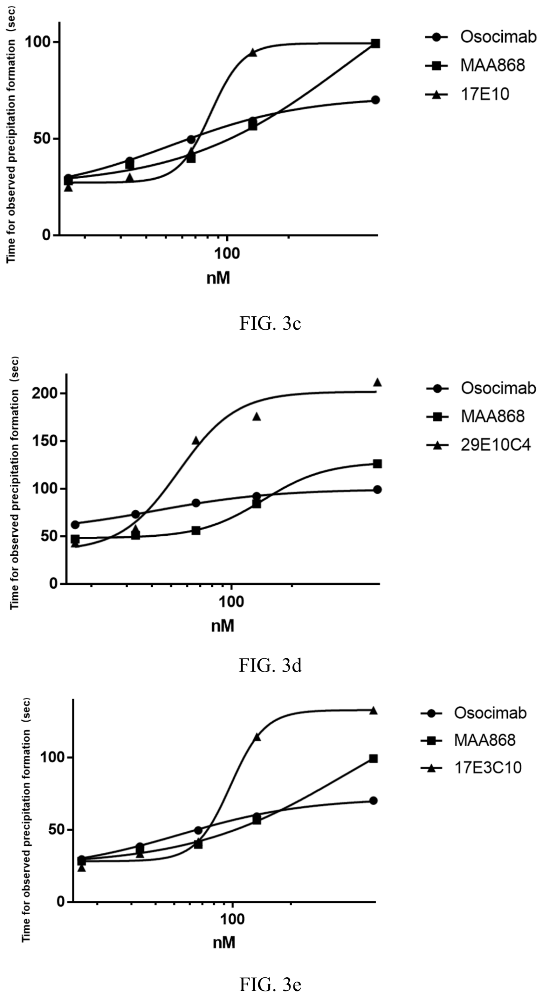

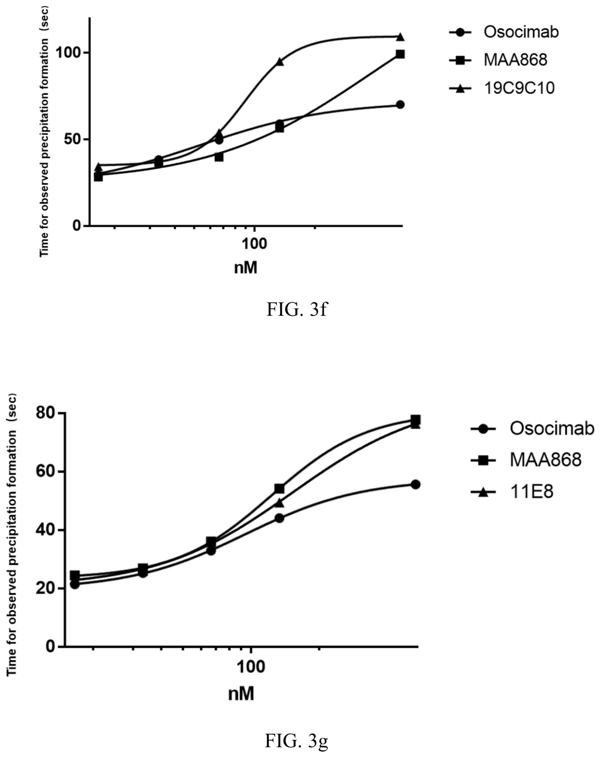

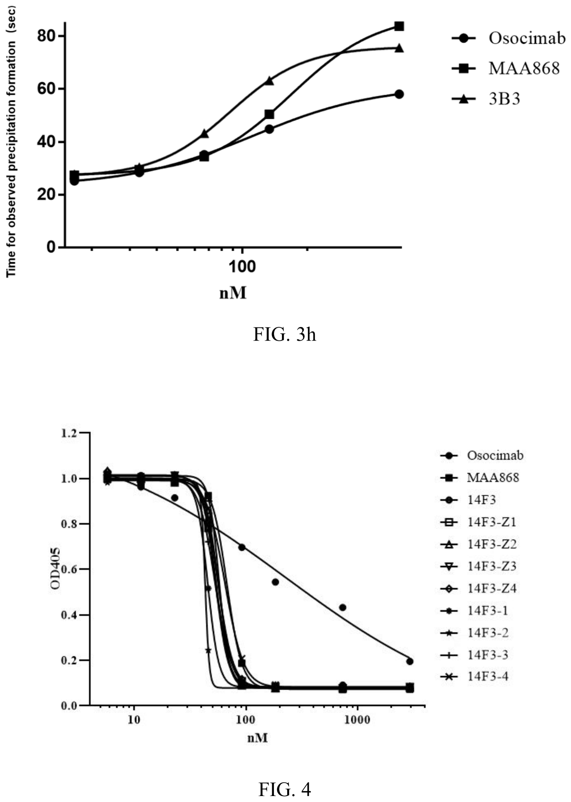

FIGs. 3a-3h show the human plasma APTT prolongation assay results for chimeric antibodies 24B6, 14F3, 17E10, 29E10C4, 17E3C10, 19C9C10, 11E8, and 3B3, respectively. The x-axis represents concentration (nM), and the y-axis represents the time (sec) taken for observable precipitation.
FIG. 4 shows the S2366 activity assay results for humanized antibodies 14F3-Z1, 14F3-Z2, 14F3-Z3, 14F3-Z4, 14F3-1, 14F3-2, 14F3-3, and 14F3-4. The x-axis represents concentration (nM), and the y-axis represents absorbance at OD₄₀₅.

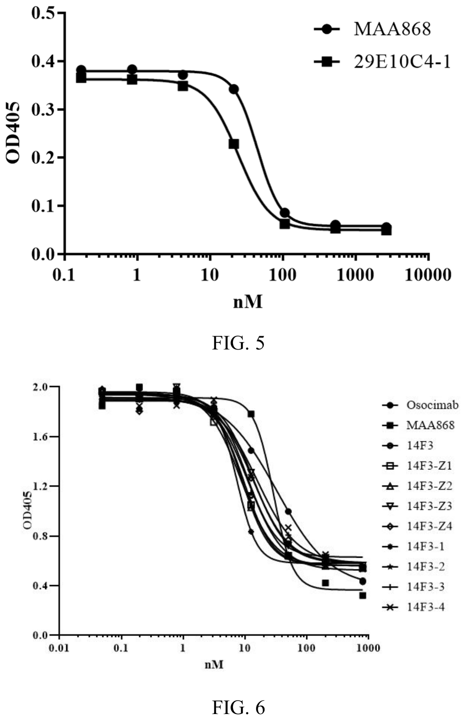

FIG. 5 shows the S2366 activity assay result for humanized antibody 29E10C4-1. The x-axis represents concentration (nM), and the y-axis represents absorbance at OD₄₀₅.
FIG. 6 shows the FXIa assay kit activity results for humanized antibodies 14F3-Z1, 14F3-Z2, 14F3-Z3, 14F3-Z4, 14F3-1, 14F3-2, 14F3-3, and 14F3-4. The x-axis represents concentration (nM), and the y-axis represents absorbance at OD₄₀₅.
FIG. 7 shows the FXIa assay kit activity results for humanized antibody 29E10C4-1 and murine antibody 29E10C4-sy. The x-axis represents concentration (nM), and the y-axis represents absorbance at OD₄₀₅.
FIG. 8 shows the human plasma APTT prolongation assay results for humanized antibodies 14F3-Z1, 14F3-Z2, 14F3-Z3, 14F3-Z4, 14F3-1, 14F3-2, 14F3-3, and 14F3-4. The x-axis represents concentration (nM), and the y-axis represents the time (sec) taken for observable precipitation.
FIG. 9 shows the human plasma APTT prolongation assay result for humanized antibody 29E10C4-1. The x-axis represents concentration (nM), and the y-axis represents the time (sec) taken for observable precipitation.
FIG. 10 shows the measured levels of free FXI in cynomolgus monkeys after administration of humanized antibody 29E10C4-1. The x-axis represents time (h/d), and the y-axis represents the relative level of free FXI compared to baseline.
FIG. 11 shows the pharmacokinetic profiles of humanized antibodies 14F3-1 and 29E10C4-1 in cynomolgus monkeys. The x-axis represents time (m/h/d), and the y-axis represents antibody concentration in plasma (µg/mL).
FIG. 12 shows the PT (prothrombin time) results in cynomolgus monkeys after administration of humanized antibodies 14F3-1 and 29E10C4-1. The x-axis represents time (m/h/d), and the y-axis represents PT (sec).

### Detailed Description of the Embodiments

The embodiments of the present invention will now be described in detail with reference to the examples. However, it should be understood by those skilled in the art that the following examples are provided solely for illustrative purposes and are not intended to limit the scope of the invention. Conditions not specifically indicated in the examples are to be carried out under conventional conditions or according to the manufacturer's instructions. Unless otherwise specified manufacturer, reagents or instruments mentioned herein are commercially available standard products.

### Abbreviations

TMB refers to 3,3',5,5'-Tetramethylbenzidine solution
PBS is Phosphate Buffer Solution.
PBST is PBS solution with Tween-20.
BSA is Bovine Serum Albumin.
IC₅₀ refers to half maximal inhibitory concentration.
Elisa refers to enzyme linked immunosorbent assay.
APTT refers to activeated partial thromboplastin time.

### Example 1: Animal Immunization and Screening of Hybridoma Cells

### Design of Target Antigen (Target Protein):

The antigen was the full-length FXI protein sequence (SEQ ID NO: 1), with a signal peptide added to the N-terminal. The C-terminal was linked via a linker peptide to an Avi-tag (a short peptide tag consisting of 15 amino acid residues), followed by another linker peptide connected to a 6xHis-tag (histidine tag), resulting in the target antigen sequence (SEQ ID NO:2). The amino acid sequence was codon-optimized for human cells and synthesized. The sequence was cloned into the multiple cloning site (MCS) of the pCDNA3.1(+) vector via HindIII/XbaI digestion. The recombinant vector was transfected into 293F cells for expression, and the eukaryotically expressed full-length FXI protein was obtained and used as the target protein for subsequent animal immunization.

### Animal Immunization:

A 1mL medical syringe was used to inject 500 µL of pH 7.2 PBS (Giboco). 1mg of the target protein was added to the PBS and mixed thoroughly. This mixture was then combined with adjuvant (Sigma Adjuvant System, SIGMA)(preheated the lyophilized powder adjuvant stored as at 4°C medical refrigerator by water bath at 42°C for 15 minutes, and set aside for later use). The combined mixture was vortexed thoroughly for 5 minutes and used for immunization. Mice were initially immunized as follows: each mouse received a dose of 200µg of the target protein (i.e., 200 µL/mouse), administered subcutaneously at two dorsal sites and intraperitoneally at two sites, with 50 µL injected per site. A second immunization was performed three weeks after the initial immunization using the same method, followed by a third immunization after another three weeks. One week after the third immunization, mouse tail vein blood was collected for titer testing. Mice with high titers were selected and immunized three days before fusion, i.e., 100 µg of the target protein diluted to 100 µL without adjuvant was injected intraperitoneally for immunization.

Titer Determination Method was as follows: 10µL of mouse tail vein blood was collected and diluted in 490µL PBS (Giboco). The solution was serially diluted 3-fold from 1:3000 up to 1:240000. 100µL of each dilution was added to Elisa plates coated with 100ng/well of the target protein, and incubated in a biochemical incubator at 37°C for 1h. Plates were washed three times with PBST using a plate washer, and residual solution was removed. 100µL of enzyme-conjugated secondary antibody diluted 1:10000 in blocking buffer was added per well and incubated in the biochemical incubator at 37°C for 1h. Plates were then washed four times with PBST and residual liquid was removed. 100µL/well of TMB substrate solution was added for color development in the dark for 8min, followed by the addition of 50µL/well of 2M H₂SO₄ to stop the reaction. OD₄₅₀ values were read using a microplate reader. P/N >2.1 is its titer.

### Hybridoma Fusion:

Mouse spleen cells and Sp2/0 mouse myeloma cells were fused using standard electrofusion techniques. Selection was performed in OPI(SIGMA)-containing HAT selection medium (DMEM medium containing 20% FBS (EeCell Bio), 1% HAT (Gibco), 1% P/S (Gibco), and 1% OPI (SIGMA)). Elisa screening was conducted on day 7 later.

### Indirect Elisa Procedure:

On the 7th day after fusion, the culture medium was replaced on the previous afternoon before Elisa testing. The medium was sucked out as much as possible without sucking out the hybridoma cells, and 180 µL of HAT selection medium (Gibco) was added. 80µL of culture supernatant was added to Elisa plates coated with the target protein, a positive control (positive mouse serum, diluted 1:1000) was set up, and plates were incubated for 1 hour at 37°C in a biochemical incubator. After washing three times with PBST, residual liquid was removed, 100µL/well of enzyme-conjugated secondary antibody (Invitrogen) diluted 1:10000 in PBST with 2% BSA was added and incubated in the biochemical incubator at 37°C for 1h. Plates were then washed four times with PBST, residual liquid was removed, and 100µL/well of TMB substrate (Solarbio) was added for color development in the dark for 8 minutes. Reactions were stopped by adding 50µL/well of 2M H₂SO₄. OD₄₅₀ values were read using a microplate reader. The positive wells were selected and transferred to a 24-well plate for further culture for two days and then were cryopreserved.

### Subcloning of Cells:

Limiting dilution was used to plate hybridoma cells at an average density of one cell per well, and positive wells with single cell clones were identified. Specifically, cells from 12-well plates were gently resuspended, and 20µL of the suspension was mixed with 20µL trypan blue by using a pipette. Cell count was determined using Counstar (cell counting software). Based on the count, 300 cells were transferred into 40mL of complete medium containing 20% FBS (EeCell Bio), 1% P/S (Gibco), 1% HAT (Gibco), 1% OPI (SIGMA), and DMEM (Gibco)). Spread 40mL of culture medium evenly onto plates at a volume of 200mL /well, using two plates in total. Incubate at 37°C in a carbon dioxide incubator. On day 5, cell morphology and the number of clones per well was examined under a microscope and labeled. On day 7, indirect Elisa was performed to detect positive wells. Positive single-cell clones were transferred to 24-well plates for further culture, then to 6-well plates after two days. Once cells reached 70-80% confluency, they were cryopreserved in two vials per clone and stored in liquid nitrogen.

### Example 2: Sequencing of Positive Hybridoma Antibody Sequences

Sequencing analysis was performed on 8 hybridoma clones targeting FXI. Details are shown in Table 1:

**Table 1: Sequences of Murine Antibody**

| Clone | Description | Sequence |
|---|---|---|
| 24B6-sy | HCDR1 | SYGMS (SEQ ID NO:3) |
| | HCDR2 | TISSGGSFTYYPDSVKG (SEQ ID NO:4) |
| | HCDR3 | LTSEGYAMEY (SEQ ID NO:5) |
| | LCDR1 | RASKSVSTSGYSHMH (SEQ ID NO:6) |
| | LCDR2 | LASNLES (SEQ ID NO:7) |
| | LCDR3 | QHSRELPYT (SEQ ID NO:8) |
| | VH | EVQLQESGGDLVKPGGSLKLSCAASGFTFSSYG MSWVRQTPDKRLEWVATISSGGSFTYYPDSVKG RFTISRDNAKNTLYLQMSSLKSEDTAMYYCASL TSEGYAMEYWGQGTSVTVSS (SEQ ID NO:51) |
| | VL | DIVLTQSPASLAVSLGQRATISCRASKSVSTSGYS HMHWYQQKLGQPPKLLIYLASNLESGVPARFSG SGSGTDFTLNIHPVEEEDAATYYCQHSRELPYTF GGGTKLEIK (SE ID NO:52) |
| 14F3-sy | HCDR1 | NYLIE (SEQ ID NO:9) |
| | HCDR2 | VINPGSGYTNYNENFKD (SEQ ID NO:10) |
| | HCDR3 | TYGKYGGAMDF (SEQ ID NO:11) |
| | LCDR1 | KASQSVSNDVA (SEQ ID NO:12) |
| | LCDR2 | YISNRYT (SEQ ID NO:13) |
| | LCDR3 | QQDYSSPPT (SEQ ID NO:14) |
| | VH | QVQLQQSGAELVRPGTSVTVSCKASGYAFSNYL IEWVNQRPGQGLEWIGVINPGSGYTNYNENFKD KATLTADTSSSTAYMQLSSLTSDDSAVYFCARTY GKYGGAMDFWGQGTSVTVSS (SEQ ID NO:53) |
| | VL | DIVMTQTPKFLLVSAGDRVTITCKASQSVSNDVA WYQQKPGQSPKLLIYYISNRYTGVPDRFTGSGY GTDFTFTINTVQAEDLAVYFCQQDYSSPPTFGGG TKLEIK (SEQ ID NO:54) |
| 17E10-sy | HCDR1 | SGFYWN (SEQ ID NO:15) |
| | HCDR2 | YISFDGRHNYNPSLKN (SEQ ID NO:16) |
| | HCDR3 | DLITTVYYYPMDY (SEQ ID NO:17) |
| | LCDR1 | KASQDVGTAVD (SEQ ID NO:18) |
| | LCDR2 | WASTRHT (SEQ ID NO: 19) |
| | LCDR3 | QQYSNYPLT (SEQ ID NO:20) |
| | VH | EVQLEESGPGLVKPSQSLSLTCSVTGYSITSGFY WNWIRQFPGNKLEWMGYISFDGRHNYNPSLKN RFSITRDTSKNQFFLKLTSVTAEDTATYYCARDLI TTVYYYPMDYWGQGTSVTVSS (SEQ ID NO:55) |
| | VL | DIVMTQSHKFMSTSVGDRVSITCKASQDVGTAV DWYQQKTGQSPKLLIYWASTRHTGVPDRFTGSG SGTDFTLTISNVQSEDLADYFCQQYSNYPLTFGA |
| | | GTKLELK (SEQ ID NO:56) |
| 29E10C 4-sy | HCDR1 | DYFIE (SEQ ID NO:21) |
| | HCDR2 | VINPGNDVTNYNEKFKG (SEQ ID NO:22) |
| | HCDR3 | SAGNYENSMDF (SEQ ID NO:23) |
| | LCDR1 | KASQSVSNDVG (SEQ ID NO:24) |
| | LCDR2 | YTSNRYT (SEQ ID NO:25) |
| | LCDR3 | QQDYHFPLT (SEQ ID NO:26) |
| | VH | QVKLEESGAELVRPGTSVKVSCKASGYAFTDYFI EWIKQRPGQGLEWIGVINPGNDVTNYNEKFKGK ATLTVDKSSTTAYMQLSSLTSDDSAVYFCTRSAG NYENSMDFWGQGTSVTVSS (SEQ ID NO:57) |
| | VL | DIVLTQSPKFLLVSAGDRVTITCKASQSVSNDVG WYQQKPGQSPKLLIYYTSNRYTGVPDRFTGSGY GTDFTFTISSVQAEDLAVYFCQQDYHFPLTFGAG TKLELK (SEQ ID NO:58) |
| 17E3C1 0-sy | HCDR1 | NYGVN (SEQ ID NO:27) |
| | HCDR2 | WINTYTGEPIYADDFKG (SEQ ID NO:28) |
| | HCDR3 | LYLYGNTYDYYAMDY (SEQ ID NO:29) |
| | LCDR1 | KASQDISTYLS (SEQ ID NO:30) |
| | LCDR2 | RANRLID (SEQ ID NO:31) |
| | LCDR3 | LQYDDLNT (SEQ ID NO:32) |
| | VH | QVKLQESGPELRKPGETVYISCKASGYTFTNYG |
| | | VNWVKQAPGKGLKWMGWINTYTGEPIYADDF KGRFAFSLETSANTAYLQISNLTNEDTATYFCARL YLYGNTYDYYAMDYWGQGTSVTVSS (SEQ ID NO:59) |
| | VL | DIVLTQTPSSMYASLGERVTITCKASQDISTYLSW FQQKPGKSPKTLIYRANRLIDGVPSRFSGSGSGQ DFSLTISSLEYEDMGVYYCLQYDDLNTFGGGTK LEIK (SEQ ID NO:60) |
| 19C9C1 0-sy | HCDR1 | DTYMH (SEQ ID NO:33) |
| | HCDR2 | RIDPANDNTKYDPKFQG (SEQ ID NO:34) |
| | HCDR3 | DYDGYAMDY (SEQ ID NO:35) |
| | LCDR1 | KASQDINKYVA (SEQ ID NO:36) |
| | LCDR2 | YTSTLEP (SEQ ID NO:37) |
| | LCDR3 | LQYDNLLT (SEQ ID NO:38) |
| | VH | EVKLEESGAELVKTGASVKLSCTASGFNIKDTY MHWVKQRPEQGLEWIGRIDPANDNTKYDPKFQ GRATVTADTSSNTAYLQLISLTSEDTAVYYCAGD YDGYAMDYWGQGTSVTVSS (SEQ ID NO:61) |
| | VL | DIVMTQTTSSLSVSLGGKVTITCKASQDINKYVA WFQHKPGKGPRLLMHYTSTLEPGIPSRFSGSGSG RDYSFTISNLEPEDIATYYCLQYDNLLTFGGGTK LEIK (SEQ ID NO:62) |
| 11E8-sy | HCDR1 | GYFMN (SEQ ID NO:39) |
| | HCDR2 | FINCYNGATSYNQKFKG (SEQ ID NO:40) |
| | HCDR3 | TISTGDSFDY (SEQ ID NO:41) |
| | LCDR1 | KASDHINNWLA (SEQ ID NO:42) |
| | LCDR2 | GASRLET (SEQ ID NO:43) |
| | LCDR3 | QQYWTTPFT (SEQ ID NO:44) |
| | VH | EVKLQESGPELVKTGASVKISCKASGYSFTGYF MNWVKQSHGKSLEWIGFINCYNGATSYNQKFK GKATFTVDASSSSAYMQFNSLTSEDSAVYYCART ISTGDSFDYWGQGSTLTVSS (SEQ ID NO:63) |
| | VL | DIVMTQSTSYLSVSLGGRVTITCKASDHINNWLA WYQQKPGDAPSLLISGASRLETGVPSRFSGSGSG KDYTLSITSLQTEDVATYYCQQYWTTPFTFGSGT KLEIK (SEQ ID NO:64) |
| 3B3-sy | HCDR1 | NYLID (SEQ ID NO:45) |
| | HCDR2 | VINPGSGITNYNEKFKG (SEQ ID NO:46) |
| | HCDR3 | GDGTYYEDFLDY (SEQ ID NO:47) |
| | LCDR1 | KASQDINSYLS (SEQ ID NO:48) |
| | LCDR2 | RANRLMD (SEQ ID NO:49) |
| | LCDR3 | LQYDEFPLT (SEQ ID NO:50) |
| | VH | EVQLEESGADLVRPGTSVKVSCKASGYAFTNYLI DWLKQRPGQGLEWIGVINPGSGITNYNEKFKGK |
| | | ATLTTDTSSSTAYMQLTNLTSDDSAVYFCARGDG TYYEDFLDYWGQGTSVTVSS (SEQ ID NO:65) |
| | VL | DIVLTQTTSSIYTSLGERVTITCKASQDINSYLSW FQQRPGRSPKTLIYRANRLMDGVPSRFSGSGSGR DFSLTISSLDYEDMGIYYCLQYDEFPLTFGAGTK LELK (SEQ ID NO:66) |

### Example 3: Activity Assay of Murine Antibodies

### S2366 Activity Assay of Monoclonal Cell Supernatants

Cryopreserved monoclonal cell lines were thawed and gradually adapted by reducing the serum content in the culture medium. Serum-free monoclonal cell lines capable of survival, along with their corresponding antibodies, were successfully obtained. The S2366 activity of the adapted antibodies was assayed as follows: FXIa was mixed with the antibody and added to a 96-well plate, followed by the addition of S2366 solution (Biocreative). The mixture was shaken to ensure homogeneity, and the optical density (OD) at 405 nm was measured. The MAA868 antibody was used as a positive control. The antibodies were each evaluated at a concentration of 20µg/mL for S2366 activity. The results of the S2366 activity assay for each antibody are shown in Table 2:

**Table 2: S2366 Activity Assay Results of Adapted Murine Antibodies**

| Antibody | OD₄₀₅ₙₘ |
|---|---|
| PBS | 1.1076 |
| MAA868 | 0.4486 |
| 24B6-sy | 0.2676 |
| 14F3-sy | 0.211 |
| 17E10-sy | 0.189 |
| 29E10C4-sy | 0.1978 |
| 17E3C10-sy | 0.4144 |
| 11E8-sy | 0.2938 |

The experimental results demonstrated that the OD405ₙₘ values of the murine antibodies to be tested were all lower than that of the positive control MAA868, indicating that the murine antibodies to be tested exhibited superior efficacy compared to the positive control antibody MAA868.

### Example 4: Preparation of Chimeric Antibodies

Based on the sequences obtained from the sequencing of hybridomas, primers were designed, and the murine antibody variable regions were cloned into the pcDNA3.1(+) vector containing the human antibody constant regions via enzyme digestion and ligation. This resulted in chimeric antibody expression vectors comprising murine antibody variable regions and human antibody constant regions. Vectors with correct sequencing results were transfected into 293F cells. The culture supernatant expressing the chimeric antibodies was collected and purified to obtain the corresponding chimeric antibodies. The chimeric antibodies are capable of avoiding the immunogenicity associated with heterologous antibodies while retaining the specific antigen-binding ability of the original antibody.

### Example 5: Activity Assay of Chimeric Antibodies

### 5.1 S2366 Activity Assay

The S2366 activity assay was performed as follows: FXIa was mixed with the antibody and added to a 96-well plate, followed by the addition of S2366 solution (Biocreative). The mixture was shaken thoroughly, and the OD value at 405nm was measured. Using MAA868 antibody as a positive control, perform S2366 assay for each antibody and calculate the IC₅₀. Both the antibodies to be tested and MAA868 were diluted with PBS starting at a concentration of 0.55mg/mL, followed by 2-fold serial dilutions to obtain 7 concentrations for the assay. The S2366 activity results for each antibody are shown in FIG.s 1a-1g, and the corresponding IC₅₀ values are listed in Table 3:

**Table 3: IC₅₀ Values from S2366 Activity Assay of Chimeric Antibodies**

| Batch | Antibody | IC₅₀(nM) from S2366 assay |
|---|---|---|
| First Batch | MAA868 | 118.2 |
| | 24B6 | 121.9 |
| | 14F3 | 78.73 |
| Second Batch | MAA868 | 83.61 |
| | 29E10C4 | 34.86 |
| | 17E3C10 | 44.38 |
| | 19C9C10 | 38.46 |
| Third Batch | MAA868 | 106.5 |
| | 11E8 | 93.08 |
| Fourth Batch | MAA868 | 64.74 |
| | 3B3 | 42.44 |

The experimental results demonstrated that the IC₅₀ values of the chimeric antibodies to be tested were either lower than or comparable to that of the positive control antibody MAA868, indicating that the chimeric antibodies to be tested exhibited superior or equivalent efficacy compared to the positive control antibody MAA868.

### 5.2 FXIa Kit Activity Assay

The FXIa activity assay was performed in accordance with the protocol provided in the FXIa kit instructions (HYPHEN BioMed, BIOPHEN^{™} Factor Xia). The MAA868 antibody was used as a positive control, and each chimeric antibody to be tested was assayed using the FXIa kit to determine its IC₅₀ value. The chimeric antibodies to be tested and MAA868 were diluted in PBS starting at an initial concentration of 2.2 mg/mL, followed by 4-fold serial dilutions to obtain 8 different concentrations for the assay. The resulting FXIa kit assay data are shown in FIG.s 2a-2g, and the corresponding IC₅₀ values are presented in Table 4:

**Table 4: IC₅₀ Values of Chimeric Antibodies from FXIa Kit Activity Assay**

| Batch | Antibody | FXIa Kit Activity Assay (nM) |
|---|---|---|
| First Batch | MAA868 | 11.18 |
| | 24B6 | 6.695 |
| | 14F3 | 7.774 |
| | 17E10 | 11.61 |
| Second Batch | MAA868 | 11.67 |
| | 29E10C4 | 0.2189 |
| | 17E3C10 | 0.7811 |
| | 19C9C10 | 1.312 |
| Third Batch | MAA868 | 13.28 |
| | 11E8 | 7.804 |

The experimental results demonstrated that the IC₅₀ values of all antibodies to be tested were either lower than or comparable to that of the positive control MAA868. In particular, antibodies 29E10C4 and 17E3C10 exhibited significantly lower IC₅₀ values than the positive control, indicating that the antibodies to be tested exhibited superior or equivalent efficacy compared to the positive control MAA868.

### 5.3 APTT Prolongation Assay in Human Plasma

The anticoagulant activity of the antibodies to be tested was evaluated using human plasma (Allcells) and an activated partial thromboplastin time (APTT) assay kit (Leagene), following the manufacturer's instructions. The MAA868 and Osocimab antibodies were used as positive controls for the APTT functional assay of chimeric antibodies. The chimeric antibodies to be tested and the positive control antibodies were diluted in PBS starting at an initial concentration of 0.8mg/mL, followed by 4-fold serial dilutions to obtain six different concentrations for the assay.

The assay results are shown in FIG.s 3a-3h. The results demonstrated that APTT values increased to varying degrees with increasing antibody concentrations. Compared with the positive control antibodies, the antibodies to be tested showed good APTT prolongation. Antibodies 24B6, 17E10, and 17E3C10 to be tested exhibited shorter clotting times than the control antibodies at lower concentrations but longer clotting times at higher concentrations. Antibodies 14F7, 29E10C4, and 19C9C10 showed clotting times similar to those of the positive controls at lower concentrations, but longer clotting times at higher concentrations. Antibodies 11E8 and 3B3 exhibited clotting times comparable to those of the positive control antibodies.

### Example 6: Humanization of Chimeric Antibodies

The selected high-activity antibodies 14F3 and 29E10C4 were subjected to humanization. Using computer modeling techniques, human germline antibody sequences were selected based on sequence homology between the murine and human antibodies, and complementarity-determining region (CDR) grafting was performed. Based on the three-dimensional structure of the murine antibodies, key amino acid residues in the framework (FR) regions of the humanized antibodies were subjected to back-mutation or non-back-mutaion as human residues, thereby yielding humanized antibody molecules with high humanization levels and excellent activity.

The sequences of the humanized antibodies constant region are shown as SEQ ID NO:85 and SEQ ID NO:86.
Sequence of Heavy chain constant region: 
ASTKGPSVFPLAPSSKSTSGGTAALGCLVKDYFPEPVTVSWN
SGALTSGVHTFPAVLQSSGLYSLSSVVTVPSSSLGTQTYICNVNHKP
SNTKVDKRVEPKSCDKTHTCPPCPAPELLGGPSVFLFPPKPKDTLM
ISRTPEVTCVVVAVSHEDPEVKFNWYVDGVEVHNAKTKPREEQY
NSTYRVVSVLTVLHQDWLNGKEYKCKVSNKALAAPIEKTISKAK
GQPREPQVYTLPPSREEMTKNQVSLTCLVKGFYPSDIAVEWESNG
QPENNYKTTPPVLDSDGSFFLYSKLTVDKSRWQQGNVFSCSVMHE
ALHNHYTQKSLSLSPGK (SEQ ID NO:85)
Sequence of Light chain constant region:



 (SEQ ID NO:86)

The humanized antibody sequence of monoclonal antibody 14F3 is shown in Table 5:

**Table 5. Humanized antibody sequence of monoclonal antibody 14F3**

| Clone | Description | Sequence |
|---|---|---|
| 14F3-1 | VH | QVQLVQSGAEVKKPGASVKVSCKASGYTFTNYLI EWVRQAPGQGLEWMGVINPGSGYTNYNENFKD RVTMTRDTSISTAYMELSRLRSDDTAVYYCARTY GKYGGAMDFWGQGTTVTVSS (SEQ ID NO:67) |
| | VL | DIVMTQSPDSLAVSLGERATINCKASQSVSNDVA WYQQKPGQPPKLLIYYISNRYTGVPDRFSGSGSG TDFTLTISSLQAEDVAVYYCQQDYSSPPTFGGGTK LEIK (SEQ ID NO:68) |
| 14F3-2 | VH | QVQLVQSGAEVKKPGASVKVSCKASGYTFTNYLI EWVRQAPGQGLEWMGVINPGSGYTNYNENFKD RVTMTRDTSISTAYMELSRLRSDDTAVYYCARTY GKYGGAMDFWGQGTTVTVSS (SEQ ID NO:69) |
| | VL | DIVMTQSPDSLAVSLGERATINCKASQSVSNDVA WYQQKPGQPPKLLIYYISNRYTGVPDRFSGSGSG TDFTLTISSLQAEDVAVYFCQQDYSSPPTFGGGTK LEIK (SEQ ID NO:70) |
| 14F3-3 | VH | QVQLVQSGAEVKKPGASVKVSCKASGYTFTNYLI EWVRQAPGQGLEWIGVINPGSGYTNYNENFKDR ATLTADTSISTAYMELSRLRSDDTAVYFCARTYGK YGGAMDFWGQGTTVTVSS (SEQ ID NO:71) |
| | VL | DIVMTQSPDSLAVSLGERATINCKASQSVSNDVA WYQQKPGQPPKLLIYYISNRYTGVPDRFSGSGSG TDFTLTISSLQAEDVAVYYCQQDYSSPPTFGGGTK LEIK（SEQ ID NO:72） |
| 14F3-4 | VH | QVQLVQSGAEVKKPGASVKVSCKASGYTFTNYLI EWVRQAPGQGLEWIGVINPGSGYTNYNENFKDR ATLTADTSISTAYMELSRLRSDDTAVYFCARTYGK |
| | | YGGAMDFWGQGTTVTVSS (SEQ ID NO: 73) |
| | VL | DIVMTQSPDSLAVSLGERATINCKASQSVSNDVA WYQQKPGQPPKLLIYYISNRYTGVPDRFSGSGSG TDFTLTISSLQAEDVAVYFCQQDYSSPPTFGGGTK LEIK (SEQ ID NO:74) |
| 14F3-Z1 | VH | QVQLVQSGAEVKKPGASVKVSCKASGYAFSNYLI EWVGQAPGQGLEWMGVINPGSGYTNYNENFKD RVTMTAHTSISTAYMELSRLRSDDTAVYFCARTYG KYGGAMDFWGQGTLVTVSS (SEQ ID NO:75) |
| | VL | DIQMTQSPSSLSASVGDRVTITCKASQSVSNDVA WYQQKPGKAPKLLIYYISNRYTGVPSRFSGSGSG TDFTFTISSLQPEDIATYFCQQDYSSPPTFGQGTKL EIK（SEQ ID NO:76） |
| 14F3-Z2 | VH | QVQLVQSGAEVKKPGASVKVSCKASGYAFSNYLI EWVGQAPGQGLEWMGVINPGSGYTNYNENFKD RVTMTRHTSISTAYMELSRLRSDDTAVYFCARTY GKYGGAMDFWGQGTLVTVSS (SEQ ID NO:77) |
| | VL | DIQMTQSPSSLSASVGDRVTITCKASQSVSNDVA WYQQKPGKAPKLLIYYISNRYTGVPSRFSGSGSG TDFTFTISSLQPEDIATYYCQQDYSSPPTFGQGTKL EIK（SEQ ID NO:78） |
| 14F3- | VH | QVQLVQSGAEVKKPGSSVKVSCKASGYAFSNYLI |
| Z3 | | EWVRQAPGQGLEWMGVINPGSGYTNYNENFKD RVTITADESTSTAYMELSSLRSEDTAVYYCARTYG KYGGAMDFWGQGTTVTVSS (SEQ ID NO:79) |
| | VL | DIQMTQSPSFLSASVGDRVTITCKASQSVSNDVA WYQQKPGKAPKLLIYYISNRYTGVPSRFSGSGSG TDFTLTISSLQPEDFATYYCQQDYSSPPTFGQGTKL EIK (SEQ ID NO:80) |
| 14F3-Z4 | VH | QVQLVQSGAEVKKPGSSVKVSCKASGYAFSNYLI EWVRQAPGQGLEWMGVINPGSGYTNYNENFKD RVTITADESTSTAYMELSSLRSEDTAVYFCARTYG KYGGAMDFWGQGTTVTVSS (SEQ ID NO:81) |
| | VL | DIQMTQSPSFLSASVGDRVTITCKASQSVSNDVA WYQQKPGKAPKLLIYYISNRYTGVPSRFSGSGSG TDFTLTISSLQPEDFATYFCQQDYSSPPTFGQGTKL EIK（SEQ ID NO:82） |

The humanized antibody sequence of monoclonal antibody 29E10C4 is shown in Table 6:

**Table 6. Humanized antibody sequence of monoclonal antibody 29E10C4**

| Clone | Descrip tion | Sequence |
|---|---|---|
| 29E10C | VH | QVQLVQSGAEVKKPGSSVKVSCKASGGTFSDYFIE |
| 4-1 | | WVRQAPGQGLEWMGVINPGNDVTNYNEKFKGRV TITADKSTSTAYMELSSLRSEDTAVYYCARSAGNYE NSMDFWGQGTTVTVSS (SEQ ID NO:83) |
| | VL | EIVLTQSPATLSLSPGERATLSCKASQSVSNDVGWY QQKPGQAPRLLIYYTSNRYTGIPARFSGSGSGTDFTL TISSLEPEDFAVYYCQQDYHFPLTFGGGTKVEIK (SEQ ID NO:84) |

### Example 7: Activity Assays of Humanized Antibodies

### 7.1 S2366 Activity Assay

The S2366 activity assay was performed as follows: FXIa factor was mixed with the antibody, added to a 96-well plate, followed by the addition of S2366 solution (Biocreative). After shaking to homogenize, the OD value at 405 nm was measured. Using Osocimab and MAA868 antibodies as positive controls, S2366 detection was performed on the antibodies and IC₅₀ values were calculated. The antibody to be tested and MAA868 were diluted with PBS starting at an initial concentration of 2.2 mg/mL, followed by 4-fold serial dilutions to obtain 8 different concentrations for the assay. The S2366 detection results of each antibody are shown in FIG.s 4-5, See Tables 7-8 for IC₅₀ values:

**Table 7: IC₅₀ values of humanized antibody of antibody 14F3 from S2366 assay**

| Antibodies | IC₅₀ (nM) from S2366 Assay |
|---|---|
| Osocimab | 226 |
| MAA868 | 67.29 |
| 14F3-Z1 | 54.07 |
| 14F3-Z2 | 57.33 |
| 14F3-Z3 | 56.33 |
| 14F3-Z4 | 55.81 |
| 14F3-1 | 45.38 |
| 14F3-2 | 42.96 |
| 14F3-3 | 52.7 |
| 14F3-4 | 64.68 |

The experimental results showed that IC₅₀ values of 8 humanized antibodies to be tested were lower than that of the positive controls, especially the IC₅₀ values of antibodies 14F3-1 and 14F3-2 were lowest, 45.38nM and 42.96nM respectively, therefore the antibodies to be tested were more effective than the positive control antibodies: Osocimab and MAA868.

**Table 8: IC₅₀ values of humanized antibody of antibody 29E10C4 from S2366 assay**

| Antibodies | IC₅₀(nM) from S2366 Assay |
|---|---|
| MAA868 | 44.94 |
| 29E10C4-1 | 24.5 |

The experimental results showed that the IC₅₀ value of humanized antibody 29E10C4-1 to be tested was significantly lower than that of positive control antibody MAA868, therefore the antibody to be tested was more effective than the positive control antibody MAA868.

### 7.2 FXIa Kit Activity Assay

FXIa kit activity assay was performed according to the manufacturer's instructions of the FXIa kit (HYPHEN BioMed, BIOPHEN^{™} Factor Xia) . Using Osocimab and MAA868 antibodies as positive controls, FXIa kit assaywas performed on each antibody to be tested and IC₅₀ values were calculated. The humanized antibody to be tested and MAA868 were diluted with PBS starting at an initial concentration of 2.2 mg/mL, followed by 4-fold serial dilutions to obtain 8 different concentrations for the assay. The FXIa kit assay results are shown in FIG.s 6-7. See Tables 9-10 for IC₅₀ values:

**Table 9: IC₅₀ values of humanized antibodies of antibody 14F3 from FXIa kit assay**

| Antibodies | FXIa kit Activity Assay (nM) |
|---|---|
| Osocimab | 32.47 |
| MAA868 | 28.85 |
| 14F3-Z1 | 8.532 |
| 14F3-Z2 | 8.793 |
| 14F3-Z3 | 13.51 |
| 14F3-Z4 | 13.6 |
| 14F3-1 | 7.495 |
| 14F3-2 | 9.689 |
| 14F3-3 | 10.7 |
| 14F3-4 | 15.74 |

The experimental results showed IC₅₀ values of each antibody to be tested were lower than that of the positive controls: Osocimab and MAA868, especially the IC₅₀ values of antibodies 14F3-1, 14F3-Z1 and 14F3-Z2 were significantly lower than that of the positive controls, therefore the antibodies to be tested were more effective than the positive control antibodies.

**Table 10: IC₅₀ values of humanized antibodies of antibody 29E10C4 from FXIa kit assay**

| Antibodies | FXIa kit Activity Assay (nM) |
|---|---|
| MAA868 | 12.69 |
| 29E10C4-sy | 0.248 |
| 29E10C4-1 | 0.5448 |

The experimental results showed the IC₅₀ values of the antibodies to be tested were significantly lower than that of MAA868, therefore the antibodies to be tested were more effective than the positive control antibody.

### 7.3 APTT Prolongation Activity Assay

### (1) APTT kit Assay for Humanized Antibodies of Antibody 14F3

Human plasma (AllCells) and activated partial thrombin time (APTT) detection kit (Leagene Biotechnology) were used to detect the anticoagulant activity of the antibody to be tested according to the manufacturer's instructions. Using MAA868 and Osocimab antibodies as positive controls, humanized antibodies were tested for APTT function. The antibody to be tested and the positive control antibody were diluted with PBS starting at an initial concentration of 0.8 mg/mL, followed by 4-fold serial dilutions to obtain 6 different concentrations for the APTT assay.

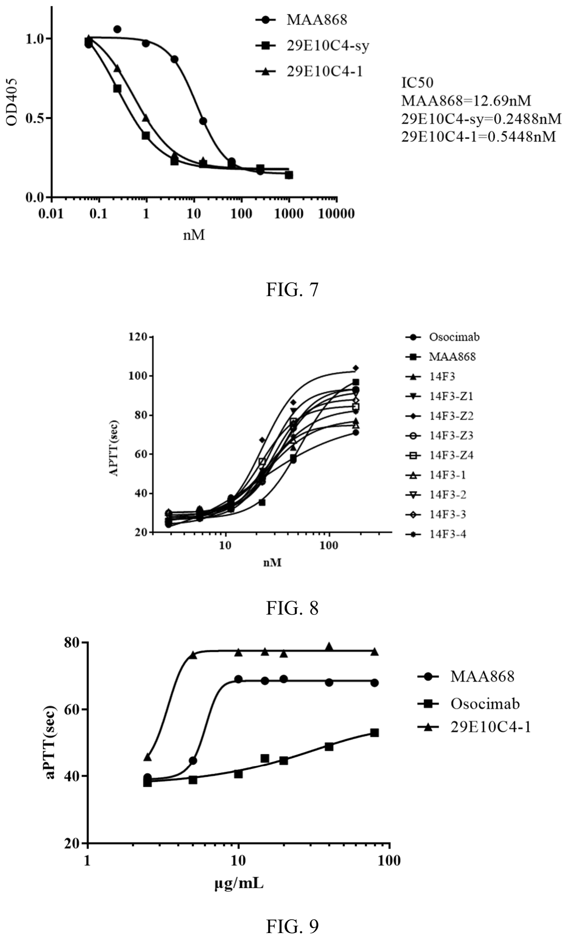

The test results are shown in FIG. 8. It can be seen from the results that as the antibody concentration increases, the APTT time increases to varying degrees. The 14F3 humanized antibody can prolong the coagulation time better than the positive control antibody at low concentrations. At high concentrations, the positive control antibody MAA868 is more effective than the 14F3 humanized antibody. The activity of the modified humanized antibodies in prolonging the coagulation time of human plasma has not been significantly reduced, and has reached a level comparable to that of the positive control antibody MAA868.

### (2) Prolongation Activity Assay for Humanized Antibodies of Antibody 29E10C4

The humanized antibody to be tested and the positive control were diluted with PBS at concentrations of 80 µg/mL, 40 µg/mL, 20 µg/mL, 15 µg/mL, 10 µg/mL, 5 µg/mL, 2.5 µg/mL, 0µg/mL, APTT assay was performed at a total of 8 concentrations . Fresh human plasma was used, corresponding concentrations of antibodies were added, and the APTT assay was performed by entrusting an outsourcing company and usinga fully automatic coagulation analyzer. The experimental results are shown in FIG. 9.

In summary, in human plasma APTT assay, the APTT prolonging effect of humanized antibody 29E10C4-1 was stronger than that of the positive control antibody MAA868.

### 7.4 Biacore affinity determination

The ability of the humanized antibody to specifically bind to the antigen was determined by using Biacore, the curve was fitted based on the experimental results, and KD, Kd, and Ka were calculated. Osocimab and MAA868 antibodies were used as positive controls to detect the affinity of the positive controls and humanized antibodies to the human FXIa factor. 7 concentrations were obtained starting at the maximum antigen concentration, followed by 2-fold serial dilutions, plus 0 concentration, for a total of 8 concentrations for biacore affinity determination. The Biacore 8K software was used to fit the curve. The KD, Kd, and Ka results of the antibodies to be tested and the positive control are shown in Table 11:

**Table 11: Biacore Results of Humanized Antibodies**

| Antibodies | ka(1/Ms) | kd(1/s) | KD (M) |
|---|---|---|---|
| MAA868 | 1.18E+06 | 8.48E-06 | 7.17E-12 |
| Osocimab | 6.45E+05 | 4.11E-03 | 6.37E-09 |
| 14F3-1 | 1.30E+07 | 1.05E-05 | 8.09E-13 |
| 29E10C4-1 | 1.35E+07 | 1.96E-05 | 1.45E-12 |

Experimental results showed that the affinity of the humanized antibody to human FXIa factor was stronger than that of the positive control, showing stronger affinity.

### 7.5 Efficacy experiment of humanized antibodies in cynomolgus monkeys

Humanized antibodies 14F3-1 and 29E10C4-1 were chosen to be administered to cynomolgus monkeys (Junke Zhengyuan). Blood was collected from cynomolgus monkeys 30 minutes before administration, and 3 mg/kg of antibody was administered intravenously. Plasma was collected at 15 minutes, 1 hour, 3 hours, 6 hours, 24 hours, 48 hours, 72 hours, 96 hours, 7 days, 10 days, and 14 days after administration for subsequent test. At the same time, fecal occult blood was detected before administration, 4 days, 7 days, and 14 days after administration to determine the safety of the drug.

After the antibody enters the blood, it inhibits the activity of FXI by binding to the antigen FXI, while the free antigen part that does not bind to the antibody can still exhibit its original activity. Therefore, by detecting the concentration of free FXI in plasma at various time points after injection of the antibody, the efficacy of the antibody in the plasma can be reflected.

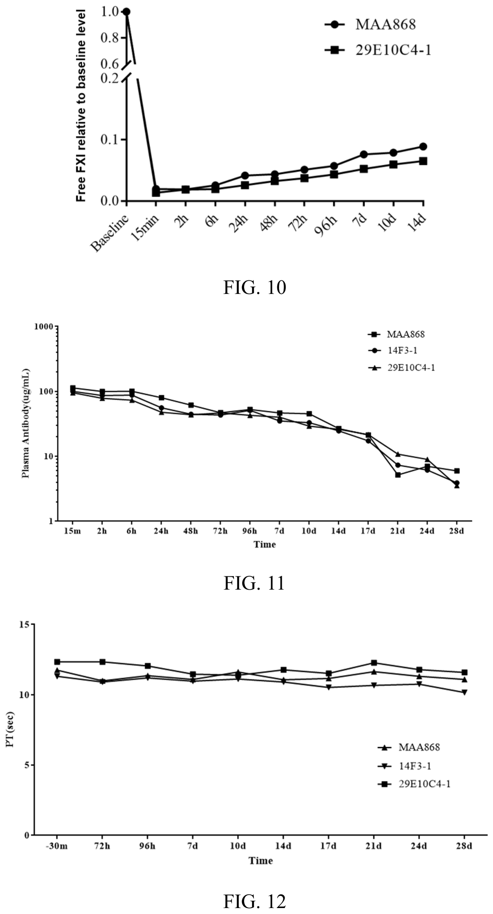

The detection method for free FXI was to use the same antibody as the injected antibody to coat the well plate, then diluted plasma was added, and then a mouse antibody with a different epitope from the antibody was used for subsequent ELISA detection. The detection results are shown in FIG. 10.

As shown in the figure, compared with the positive control antibody MAA868, the humanized antibody 29E10C4-1 exhibited a stronger inhibitory effect on free FXI than MAA868.

Since the biggest safety issue of anticoagulants was bleeding, and blood cells could be detected in the feces after minor gastrointestinal bleeding, feces were collected on -3d, 4d, 7d, 10d, and 14d before and after the injection in cynomolgus monkeys to conduct fecal occult blood test. After testing fecal color, texture, fat globules, red blood cells, white blood cells and other indicators, no abnormalities were detected in the fecal occult blood for MAA868 and humanized antibody 29E10C4-1.

In summary, the activity of humanized antibody 29E10C4-1 was better than that of the positive control MAA868, and it showed better safety.

### 7.6 Pharmacokinetics experiment (PK) of humanized antibody in cynomolgus monkey

Following the steps below to detect the pharmacokinetic indicators of humanized antibodies 14F3-1 and 29E10C4-1 in cynomolgus monkeys:
(1) Injection in cynomolgus monkey (United-power):
   Administration method: intravenous injection, dosage: 3mg/kg;
(2) Blood collection point setting:
   -30min, 15min, 2h, 6h, 24h, 48h, 72h, 96h, 7d, 10d, 14d, 17d, 21d, 24d, 28d, 31d, 35d blood collection point;
(3) Blood collection plan:
   About 1 mL of whole blood sample was collected from the veins of the non-administration limb of the cynomolgus monkey, immediately put into a vacuum blood collection tube with a marked sample number and anticoagulated with sodium citrate 9:1, placed in an ice box for storage, and placed in a centrifuge at 2 to 8°C and centrifuged at 3000 rpm for 10 minutes, within 1 hour after collection. The supernatant was transferred into an EP tube marked with a sample number. After processed, the samples were stored in a refrigerator at -60~-90°C;
(4) Sample processing: 5 µL of plasma was dissolved in 45 µL of PBS, centrifuged at 200 g for 10 min at 4°C, the red blood cells were discarded, and the serum was collected and cryopreserved at -80°C for subsequent PK test;
(5) Coating antibody: Specific protein F11 was diluted to 1ug/mL with coating buffer 1×PBS, 100µL of diluted protein F11 was added to each well (costar 96-well plate), and incubated overnight at 4°C;
(6) Blocking: The coating solution in the 96-well plate was discarded and the plate was patted dry. 200 µL of 2% BSA (diluted in PBST, m/v) was added to each well, and blocked at 37°C for 2 hours;
(7) Primary antibody incubation: The coating solution was discarded and the plate was patted dry. 100 µL of the corresponding standard curve samples and serum samples were added to each well, and incubated at 37°C for 1 hour. The standard curve samples MAA868, 14F3-1 and 29E10C4-1 and serum samples were all diluted with 2% BSA. The concentration gradients of the standard curve samples MAA868, 14F3-1 and 29E10C4-1 were all 0ng/mL, 1ng/mL and 2ng/mL, 4ng/mL, 8ng/mL, 16ng/mL, 32ng/mL, 64ng/mL, 128ng/mL, 256ng/mL and 512ng/mL; the 15min, 30min, 1h, 3h, 8h, 24h, 48h, 96h and 168h samples of serum samples MAA868, 14F3-1 and 29E10C4-1 were all diluted at two ratios: 100× and 1000×.
(8) Washing: The coating solution was removed and each well was washed 3 times with 200 µL washing buffer PBST (comprising 0.05% Tween-20);
(9) Secondary antibody incubation: 100 µL of Mouse Anti-Human IgG Fc Antibody [HRP] (Genscript) (diluted with 2% BSA at 1:10000) was added, and incubated at 37°C for 1 hour;
(10) Washing: The secondary antibody was removed and each well was washed 4 times with 200 µL washing buffer PBST (comprising 0.05% Tween-20);
(11) TMB color development and termination: 100 µL of TMB (Solarbio) was added for color development for 6 minutes, followed by the addition of 2M sulfuric acid to terminate the reaction;
(13) Reading: The 96-well plate was placed in a microplate reader and the absorbance value at 450nm was read;
(14) Data processing: Curves were plotted, the antibody concentration at each time point was calculated, and DAS2.0 software was used to calculate the PK parameters.

The test results are shown in FIG. 11. It can be seen that the half-lives of humanized antibodies 14F3-1 and 29E10C4-1 are more than 200 hours, showing similar pharmacokinetics to the control antidoby MAA868.

### 7.7 Bleeding Risk Detection for Humanized Antibody in Cynomolgus Monkeys

In order to detect the bleeding risk for humanized antibodies in cynomolgus monkeys, first, whether the antibodies 14F3-1 and 29E10C4-1 affect exogenous coagulation was detected, fresh plasma was collected according to the method in section 7.5, and provided to a third party for detection of exogenous coagulation indicator PT (plasma prothrombin time), following conventional methods known to those skilled in the art.

The experimental results are shown in FIG. 12. It can be seen that in the PT (plasma prothrombin time) test after administration in cynomolgus monkeys, similar to MAA868, neither 14F3-1 nor 29E10C4-1 affects the PT test value in cynomolgus monkeys. Therefore, these two antibodies do not affect the extrinsic coagulation pathway and do not increase the risk of bleeding caused by inhibition of the extrinsic pathway.

In addition, the fecal occult blood of cynomolgus monkeys was detected before administration and on days 4, 7, 14, 21, 28, and 35 days after administration. The specific detection indicators were: feces color, feces properties, fat globules, red blood cells, white blood cells, and parasite eggs and parasites, etc. The result was that no abnormalities were detected in the fecal occult blood for MAA868 and humanized antibodies 14F3-1 and 29E10C4-1. Therefore, the two antibodies 14F3-1 and 29E10C4-1 exhibited a low risk of gastrointestinal bleeding.

The present disclosure has been described through the above-mentioned embodiments, but it should be understood that the above-mentioned embodiments are only for the purpose of illustration, and are not intended to limit the present disclosure to the scope of the described embodiments. In addition, those skilled in the art can understand that the present disclosureis not limited to the above embodiments, and more variations and modifications can be made according to the teachings of the present disclosure, and these variations and modifications all fall within the scope of protection claimed by the present disclosure. The protection scope of the present disclosure is defined by the appended claims and their equivalent scope.

## Claims

1. An antibody or antigen-binding fragment thereof, which specifically binds to FXI and/or FXIa, comprising a heavy chain variable region and/or a light chain variable region, wherein:
1) the heavy chain variable region comprises:
(i) three heavy chain complementarity determining regions (HCDRs) HCDR1, HCDR2, and HCDR3, contained within a peptide segment with an amino acid sequence of SEQ ID NO: 51, 53, 55, 57, 59, 61, 63, 65, 67, 69, 71, 73, 75, 77, 79, 81, or 83; or
(ii) a sequence that collectively comprises at least one but no more than 5 amino acid mutations (preferably amino acid substitutions, preferably conservative substitutions) on the three heavy chain complementarity-determining regions (HCDRs), relative to the sequences of the three heavy chain complementarity-determining regions (HCDRs) defined in (i);
and/or
2) the light chain variable region comprises:
(i) three light chain complementarity determining regions (LCDRs) LCDR1, LCDR2, and LCDR3, contained within a peptide segment with an amino acid sequence of SEQ ID NO: 52, 54, 56, 58, 60, 62, 64, 66, 68, 70, 72, 74, 76, 78, 80, 82, or 84; or
(ii) a sequence that collectively comprises at least one but no more than 5 amino acid mutations (preferably amino acid substitutions, preferably conservative substitutions) on the three light chain complementarity determining regions (LCDRs), relative to the sequences of the three light chain complementarity determining regions (LCDRs) defined in (i).

2. The antibody or antigen-binding fragment thereof according to claim 1, comprising:
three heavy chain complementarity determining regions (HCDRs) HCDR1, HCDR2, and HCDR3, contained within a peptide segment with an amino acid sequence of SEQ ID NO: 51, 53, 55, 57, 59, 61, 63, 65, 67, 69, 71, 73, 75, 77, 79, 81, or 83; and/or
three light chain complementarity determining regions (LCDRs) LCDR1, LCDR2, and LCDR3, contained within a peptide segment with an amino acid sequence of SEQ ID NO: 52, 54, 56, 58, 60, 62, 64, 66, 68, 70, 72, 74, 76, 78, 80, 82, or 84.

3. An antibody or antigen-binding fragment thereof, which specifically binds to FXI and/or FXIa, comprising:
a) a heavy chain complementarity determining region HCDR3, wherein the HCDR3 comprises or consists of any one amino acid sequence selected from the group consisting of the following amino acid sequences: SEQ ID NO: 5, 11, 17, 23, 29, 35, 41, and 47, or the HCDR3 comprises an amino acid sequence having 1, 2, or 3 amino acid mutations (preferably amino acid substitutions, preferably conservative substitutions) relative to the amino acid sequences as set forth in SEQ ID NO: 5, 11, 17, 23, 29, 35, 41, and 47; and
b) a light chain complementarity determining region LCDR3, wherein the LCDR3 comprises or consists of any one amino acid sequence selected from the group consisting of the following amino acid sequences: SEQ ID NO: 8, 14, 20, 26, 32, 38, 44, and 50, or the LCDR3 comprises an amino acid sequence having 1, 2, or 3 amino acid mutations (preferably amino acid substitutions, preferably conservative substitutions) relative to the amino acid sequences as set forth in SEQ ID NO: 8, 14, 20, 26, 32, 38, 44 and 50.

4. The antibody or antigen-binding fragment thereof according to claim 3, further comprising:
c) a heavy chain complementarity determining region HCDR1, wherein the HCDR1 comprises or consists of any one amino acid sequence selected from the group consisting of the following amino acid sequences: SEQ ID NO: 3, 9, 15, 21, 27, 33, 39, and 45, or the HCDR1 comprises an amino acid sequence having 1, 2, or 3 amino acid mutations (preferably amino acid substitutions, preferably conservative substitutions) relative to the amino acid sequence as set forth in SEQ ID NO: 3, 9, 15, 21, 27, 33, 39 or 45;
d) a heavy chain complementarity determining region HCDR2, wherein the HCDR2 comprises or consists of any one amino acid sequence selected from the group consisting of the following amino acid sequences: SEQ ID NO: 4, 10, 16, 22, 28, 34, 40, and 46, or the HCDR2 comprises an amino acid sequence having 1, 2, or 3 amino acid mutations (preferably amino acid substitutions, preferably conservative substitutions) relative to the amino acid sequence as set forth in SEQ ID NO: 4, 10, 16, 22, 28, 34, 40 or 46;
e) a light chain complementarity determining region LCDR1, wherein the LCDR1 comprises or consists of any one amino acid sequence selected from the group consisting of the following amino acid sequences: SEQ ID NO: 6, 12, 18, 24, 30, 36, 42, and 48, or the LCDR1 comprises an amino acid sequence having 1, 2, or 3 amino acid mutations (preferably amino acid substitutions, preferably conservative substitutions) relative to the amino acid sequence as set forth in SEQ ID NO: 6, 12, 18, 24, 30, 36, 42 or 48; and
f) a light chain complementarity determining region LCDR2, wherein the LCDR2 comprises or consists of any one amino acid sequence selected from the group consisting of the following amino acid sequences: SEQ ID NO: 7, 13, 19, 25, 31, 37, 43, and 49, or the LCDR2 comprises an amino acid sequence having 1, 2, or 3 amino acid mutations (preferably amino acid substitutions, preferably conservative substitutions) relative to the amino acid sequence as set forth in SEQ ID NO: 7, 13, 19, 25, 31, 37, 43, or 49.

5. An antibody or antigen-binding fragment thereof, comprising three heavy chain complementarity determining regions (HCDRs) HCDR1, HCDR2, and HCDR3; and three light chain complementarity determining regions (LCDRs) LCDR1, LCDR2, and LCDR3, wherein:
the HCDR1 comprises or consisting of the amino acid sequence of SEQ ID NO: 3, 9, 15, 21, 27, 33, 39, or 45, or the HCDR1 comprises an amino acid sequence having 1, 2, or 3 amino acid mutations (preferably amino acid substitutions, preferably conservative substitutions) relative to the amino acid sequence as set forth in SEQ ID NO: 3, 9, 15, 21, 27, 33, 39, or 45;
the HCDR2 comprises or consisting of the amino acid sequence of SEQ ID NO: 4, 10, 16, 22, 28, 34, 40, or 46, or the HCDR2 comprises an amino acid sequence having 1, 2, or 3 amino acid mutations (preferably amino acid substitutions, preferably conservative substitutions) relative to the amino acid sequence as set forth in SEQ ID NO: 4, 10, 16, 22, 28, 34, 40, or 46;
the HCDR3 comprises or consisting of the amino acid sequence of SEQ ID NO: 5, 11, 17, 23, 29, 35, 41, or 47, or the HCDR3 comprises an amino acid sequence having 1, 2, or 3 amino acid mutations (preferably amino acid substitutions, preferably conservative substitutions) relative to the amino acid sequence as set forth in SEQ ID NO: 5, 11, 17, 23, 29, 35, 41, or 47;
the LCDR1 comprises or consisting of the amino acid sequence of SEQ ID NO: 6, 12, 18, 24, 30, 36, 42, or 48, or the LCDR1 comprises an amino acid sequence having 1, 2, or 3 amino acid mutations (preferably amino acid substitutions, preferably conservative substitutions) relative to the amino acid sequence as set forth in SEQ ID NO: 12, 18, 24, 30, 36, 42, or 48;
the LCDR2 comprises or consisting of the amino acid sequence of SEQ ID NO: 7, 13, 19, 25, 31, 37, 43, or 49, or the LCDR2 comprises an amino acid sequence having 1, 2, or 3 amino acid mutations (preferably amino acid substitutions, preferably conservative substitutions) relative to the amino acid sequence as set forth in SEQ ID NO: 7, 13, 19, 25, 31, 37, 43, or 49;
the LCDR3 comprises or consisting of the amino acid sequence of SEQ ID NO: 8, 14, 20, 26, 32, 38, 44, or 50, or the LCDR3 comprises an amino acid sequence having 1, 2, or 3 amino acid mutations (preferably amino acid substitutions, preferably conservative substitutions) relative to the amino acid sequence as set forth in SEQ ID NO: 8, 14, 20, 26, 32, 38, 44, or 50.

6. The antibody or antigen-binding fragment thereof according to claim 1 or 2, comprising:
(1) three heavy chain complementarity determining regions (HCDRs) HCDR1, HCDR2, and HCDR3, contained within a peptide segment of the amino acid sequence as set forth in SEQ ID NO: 51, and
three light chain complementarity determining regions (LCDRs) LCDR1, LCDR2, and LCDR3, contained within a peptide segment of the amino acid sequence as set forth in SEQ ID NO: 52;
(2) three heavy chain complementarity determining regions (HCDRs) HCDR1, HCDR2, and HCDR3, contained within a peptide segment of the amino acid sequence as set forth in SEQ ID NO: 53, 67, 69, 71, 73, 75, 77, 79, or 81, and
three light chain complementarity determining regions (LCDRs) LCDR1, LCDR2, and LCDR3, contained within a peptide segment of the amino acid sequence as set forth in SEQ ID NO: 54, 68, 70, 72, 74, 76, 78, 80, or 82;
(3) three heavy chain complementarity determining regions (HCDRs) HCDR1, HCDR2, and HCDR3, contained within a peptide segment of the amino acid sequence as set forth in SEQ ID NO: 55, and
three light chain complementarity determining regions (LCDRs) LCDR1, LCDR2, and LCDR3, contained within a peptide segment of the amino acid sequence as set forth in SEQ ID NO: 56;
(4) three heavy chain complementarity determining regions (HCDRs) HCDR1, HCDR2, and HCDR3, contained within a peptide segment of the amino acid sequence as set forth in SEQ ID NO: 57 or 83, and
three light chain complementarity determining regions (LCDRs) LCDR1, LCDR2, and LCDR3, contained within a peptide segment of the amino acid sequence as set forth in SEQ ID NO: 58 or 84;
(5) three heavy chain complementarity determining regions (HCDRs) HCDR1, HCDR2, and HCDR3, contained within a peptide segment of the amino acid sequence as set forth in SEQ ID NO: 59, and
three light chain complementarity determining regions (LCDRs) LCDR1, LCDR2, and LCDR3, contained within a peptide segment of the amino acid sequence as set forth in SEQ ID NO: 60;
(6) three heavy chain complementarity determining regions (HCDRs) HCDR1, HCDR2, and HCDR3, contained within a peptide segment of the amino acid sequence as set forth in SEQ ID NO: 61, and
three light chain complementarity determining regions (LCDRs) LCDR1, LCDR2, and LCDR3, contained within a peptide segment of the amino acid sequence as set forth in SEQ ID NO: 62;
(7) three heavy chain complementarity determining regions (HCDRs) HCDR1, HCDR2, and HCDR3, contained within a peptide segment of the amino acid sequence as set forth in SEQ ID NO: 63, and
three light chain complementarity determining regions (LCDRs) LCDR1, LCDR2, and LCDR3, contained within a peptide segment of the amino acid sequence as set forth in SEQ ID NO: 64; or
(8) three heavy chain complementarity determining regions (HCDRs) HCDR1, HCDR2, and HCDR3, contained within a peptide segment of the amino acid sequence as set forth in SEQ ID NO: 65, and
three light chain complementarity determining regions (LCDRs) LCDR1, LCDR2, and LCDR3, contained within a peptide segment of the amino acid sequence as set forth in SEQ ID NO: 66.

7. The antibody or antigen-binding fragment thereof according to any one of claims 1-6, comprising:
(1) a HCDR1 comprising or consisting of SEQ ID NO: 3,
a HCDR2 comprising or consisting of SEQ ID NO: 4,
a HCDR3 comprising or consisting of SEQ ID NO: 5,
a LCDR1 comprising or consisting of SEQ ID NO: 6,
a LCDR2 comprising or consisting of SEQ ID NO: 7, and
a LCDR3 comprising or consisting of SEQ ID NO: 8;
(2) a HCDR1 comprising or consisting of SEQ ID NO: 9,
a HCDR2 comprising or consisting of SEQ ID NO: 10,
a HCDR3 comprising or consisting of SEQ ID NO: 11,
a LCDR1 comprising or consisting of SEQ ID NO: 12,
a LCDR2 comprising or consisting of SEQ ID NO: 13, and
a LCDR3 comprising or consisting of SEQ ID NO: 14;
(3) a HCDR1 comprising or consisting of SEQ ID NO: 15,
a HCDR2 comprising or consisting of SEQ ID NO: 16,
a HCDR3 comprising or consisting of SEQ ID NO: 17,
a LCDR1 comprising or consisting of SEQ ID NO: 18,
a LCDR2 comprising or consisting of SEQ ID NO: 19, and
a LCDR3 comprising or consisting of SEQ ID NO: 20;
(4) a HCDR1 comprising or consisting of SEQ ID NO:21,
a HCDR2 comprising or consisting of SEQ ID NO: 22,
a HCDR3 comprising or consisting of SEQ ID NO: 23,
a LCDR1 comprising or consisting of SEQ ID NO: 24,
a LCDR2 comprising or consisting of SEQ ID NO: 25, and
a LCDR3 comprising or consisting of SEQ ID NO: 26;
(5) a HCDR1 comprising or consisting of SEQ ID NO:27,
a HCDR2 comprising or consisting of SEQ ID NO: 28,
a HCDR3 comprising or consisting of SEQ ID NO: 29,
a LCDR1 comprising or consisting of SEQ ID NO: 30,
a LCDR2 comprising or consisting of SEQ ID NO: 31, and
a LCDR3 comprising or consisting of SEQ ID NO: 32;
(6) a HCDR1 comprising or consisting of SEQ ID NO: 33,
a HCDR2 comprising or consisting of SEQ ID NO: 34,
a HCDR3 comprising or consisting of SEQ ID NO: 35,
a LCDR1 comprising or consisting of SEQ ID NO: 36,
a LCDR2 comprising or consisting of SEQ ID NO: 37, and
a LCDR3 comprising or consisting of SEQ ID NO: 38;
(7) a HCDR1 comprising or consisting of SEQ ID NO: 39,
a HCDR2 comprising or consisting of SEQ ID NO: 40,
a HCDR3 comprising or consisting of SEQ ID NO: 41,
a LCDR1 comprising or consisting of SEQ ID NO: 42,
a LCDR2 comprising or consisting of SEQ ID NO: 43, and
a LCDR3 comprising or consisting of SEQ ID NO: 44; or
(8) a HCDR1 comprising or consisting of SEQ ID NO: 45,
a HCDR2 comprising or consisting of SEQ ID NO: 46,
a HCDR3 comprising or consisting of SEQ ID NO: 47,
a LCDR1 comprising or consisting of SEQ ID NO: 48,
a LCDR2 comprising or consisting of SEQ ID NO: 49, and
a LCDR3 comprising or consisting of SEQ ID NO: 50.

8. An antibody or antigen-binding fragment thereof, which specifically binds to FXI and/or FXIa, comprising:
a heavy chain variable region, wherein the heavy chain variable region has at least 80% identity to any one amino acid sequence selected from the group consisting of the following amino acid sequences: SEQ ID NO: 51, 53, 55, 57, 59, 61, 63, 65, 67, 69, 71, 73, 75, 77, 79, 81, and 83; preferably, the heavy chain variable region is selected from an amino acid sequence having one or more (preferably no more than 10, more preferably no more than 5) amino acid mutations (preferably amino acid substitutions, more preferably amino acid conservative substitutions) relative to the amino acid sequence of SEQ ID NO: 51, 53, 55, 57, 59, 61, 63, 65, 67, 69, 71, 73, 75, 77, 79, 81, or 83; preferably, the amino acid mutations do not occur in the heavy chain complementarity determining regions; preferably, the heavy chain variable region has CDRs identical to those of SEQ ID NO: 51, 53, 55, 57, 59, 61, 63, 65, 67, 69, 71, 73, 75, 77, 79, 81, or 83, and the non-CDRs of the heavy chain variable region have a sequence with at least 80% identity to the non-CDRs of the amino acid sequence of SEQ ID NO: 51, 53, 55, 57, 59, 61, 63, 65, 67, 69, 71, 73, 75, 77, 79, 81, or 83; preferably, the heavy chain variable region has any one amino acid sequence selected from the group consisting of the following amino acid sequences: SEQ ID NO: 51, 53, 55, 57, 59, 61, 63, 65, 67, 69, 71, 73, 75, 77, 79, 81, and 83; and/or
a light chain variable region, wherein the light chain variable region has at least 80% identity to any one amino acid sequence selected from the group consisting of the following amino acid sequences: SEQ ID NO: 52, 54, 56, 58, 60, 62, 64, 66, 68, 70, 72, 74, 76, 78, 80, 82, and 84; preferably, the light chain variable region is selected from an amino acid sequence of having one or more (preferably no more than 10, more preferably no more than 5) amino acid mutations (preferably amino acid substitutions, more preferably amino acid conservative substitutions) relative to the amino acid sequence of SEQ ID NO: 52, 54, 56, 58, 60, 62, 64, 66, 68, 70, 72, 74, 76, 78, 80, 82, and 84; preferably, the amino acid mutations do not occur in the light chain complementarity determining regions; preferably, the light chain variable region has CDRs identical to those of SEQ ID NO: 52, 54, 56, 58, 60, 62, 64, 66, 68, 70, 72, 74, 76, 78, 80, 82, or 84, and the non-CDRs of the light chain variable region have a sequence with at least 80% identity to the non-CDRs of the amino acid sequence of SEQ ID NO: 52, 54, 56, 58, 60, 62, 64, 66, 68, 70, 72, 74, 76, 78, 80, 82, or 84; preferably, the light chain variable region has any one amino acid sequence selected from the group consisting of the following amino acid sequences: SEQ ID NO: 52, 54, 56, 58, 60, 62, 64, 66, 68, 70, 72, 74, 76, 78, 80, 82, and 84.

9. The antibody or antigen-binding fragment thereof according to claim 8, comprising the heavy chain variable region and the light chain variable region, wherein the amino acid sequence pair of the heavy chain variable region and light chain variable region is selected from the group consisting of the following amino acid sequence pairs: SEQ ID NO:51 and 52, SEQ ID NO:53 and 54, SEQ ID NO:55 and 56, SEQ ID NO:57 and 58, SEQ ID NO:59 and 60, SEQ ID NO:61 and 62, SEQ ID NO:63 and 64, SEQ ID NO:65 and 66, SEQ ID NO:67 and 68, SEQ ID NO:69 and 70, SEQ ID NO:71 and 72, SEQ ID NO:73 and 74, SEQ ID NO:75 and 76, SEQ ID NO:77 and 78, SEQ ID NO:79 and 80, SEQ ID NO:81 and 82, and SEQ ID NO: 83 and 84.

10. The antibody or antigen-binding fragment thereof according to claim 1 or 2, wherein the CDRs are defined by the IMGT definition, Kabat definition, Chothia definition, Abm definition, and/or Contact definition.

11. The antibody or antigen-binding fragment thereof according to any one of claims 1 to 10, further comprising a constant region derived from an IgG antibody, an IgM antibody, an IgA antibody, an IgD antibody, or an IgE antibody; preferably, the constant region is derived from an IgG1 antibody, an IgG2 antibody, an IgG3 antibody or an IgG4 antibody.

12. The antibody or antigen-binding fragment thereof according to any one of claims 1 to 11, further comprising: a heavy chain constant region comprising consisting of SEQ ID NO:85 or an amino acid sequence having at least 80%, 85%, 90%, 91%, 92%, 93%, 94%, 95%, 96%, 97%, 98% or 99% identity to the sequence of SEQ ID NO:85; and/or
a light chain constant region comprising or consisting of SEQ ID NO:86 or an amino acid sequence having at least 80%, 85%, 90%, 91%, 92%, 93%, 94%, 95%, 96%, 97%, 98% or 99% identity to the sequence of SEQ ID NO:86.

13. The antibody or antigen-binding fragment thereof according to any one of claims 1 to 12, wherein the antibody or antigen-binding fragment thereof is a murine antibody, a chimeric antibody, a humanized antibody or a human antibody.

14. The antibody or antigen-binding fragment thereof according to any one of claims 1 to 12, wherein the antibody or antigen-binding fragment thereof is selected from scFv, Fab, Fab', (Fab')2, Fv fragment, disulfide-linked Fv (dsFv) and diabody.

15. A pharmaceutical composition, comprising the antibody or antigen-binding fragment thereof according to any one of claims 1 to 14 and a pharmaceutically acceptable excipient.

16. The pharmaceutical composition according to claim 15, further comprising an additional therapeutic agent.

17. The pharmaceutical composition according to claim 16, wherein the additional therapeutic agent is selected from at least one of an antiplatelet agent, an anticoagulant agent, or a thrombolytic agent; preferably, the additional therapeutic agent is selected from at least one of aspirin, clopidogrel, prasugrel, ticagrelor, abciximab, eptifibatide, vorapaxar, unfractionated heparin, heparin, low molecular weight heparin, warfarin, fondaparinux sodium, edoxaban, betrixaban, rivaroxaban, apixaban, dabigatran etexilate, argatroban, bivalirudin, streptokinase, urokinase, alteplase, and prourokinase.

18. The pharmaceutical composition according to claim 16, wherein the additional therapeutic agent is another antibody or nucleic acid recognizing a different epitope of FXI and/or FXIa, or an antibody that specifically binds to a receptor or ligand selected from thrombin, Antiplasmin, Factor XII, Factor VIII, Factor VII, Factor X, Factor IX, Factor II, Tissue Factor, P-selectin and its ligand, L-selectin and its ligand, or any combination of the above antibodies.

19. A nucleic acid, wherein the nucleic acid has a nucleotide sequence encoding the antibody or antigen-binding fragment thereof according to any one of claims 1 to 14.

20. An expression vector, wherein the expression vector comprises the nucleic acid according to claim 19.

21. A host cell, wherein the host cell comprises the expression vector according to claim 20.

22. The host cell according to claim 21, wherein the host cell is a prokaryotic cell or a eukaryotic cell.

23. The host cell according to claim 21, wherein the host cell is *Escherichia coli,* a yeast cell, an insect cell, a plant cell or a mammalian cell.

24. The host cell according to claim 21, wherein the host cell is a Chinese hamster ovary cell (CHO), a CHO cell variant, a 293 cell, or an NS0 cell.

25. A method of manufacturing an antibody or antigen-binding fragment thereof, comprising culturing the host cell according to any one of claims 21 to 24, and recovering the antibody or antigen-binding fragment thereof expressed by the cells from the culture.

26. Use of the antibody or antigen-binding fragment thereof according to any one of claims 1 to 14 or the pharmaceutical composition according to any one of claims 15 to 18 in the preparation of a medicament for the treatment of coagulation or thromboembolism.

27. The antibody or antigen-binding fragment thereof according to any one of claims 1 to 14 or the pharmaceutical composition according to any one of claims 15 to 18, for use in the treatment of coagulation or thromboembolism.

28. A method for treating coagulation or thromboembolism, comprising administering a therapeutically effective amount of the antibody or antigen-binding fragment thereof according to any one of claims 1 to 14 or the pharmaceutical composition according to any one of claims 15 to 18 thereof.
